# EUROPEAN PATENT APPLICATION

(11) **EP 2 484 765 A1**
(43) Date of publication of application: **08.08.2012**
(21) Application number: 11153379.0
(22) Date of filing: 04.02.2011
(51) Int. Cl.: C12N 15/67, C12N 15/113

(54) **Cell transfection under rab37 deficiency or inhibition**

(71) Applicant: Universitätsmedizin der Johannes Gutenberg-Universität Mainz, 55131 Mainz (DE)
(72) Inventor: Florin, Luise, 55128 Mainz (DE); Spoden, Gilles, 65183 Wiesbaden (DE)
(74) Representative: Michalski Hüttermann & Partner

(57) **Abstract**

The present invention is related to methods for increasing endocytotic uptake efficacy of a cell, as well as to cells thus produced, and uses thereof. The method comprises a step in which cellular activity of the Rab37 gene product, or an ortholog thereof, is impaired, or inhibited

## Description

The present invention is related to methods for increasing endocytotic uptake efficacy of a cell, as well as to cells thus produced, and uses thereof.

### Introduction

Successful induction of endocytotic uptake, e.g. in a transfection or infection experiment, is a limiting factor in the development of a cell line, which is to be used for heterologous protein expression, or for vaccine production.

Generally, induced endocytosis is subject to many influences, which may affect the endocytotic uptake efficacy. In a cell transfection experiment, for instance, in which the transfection is related to endocytosis, critical factors are, among others, the amount of transfecting nucleic acid per reaction well, the amount of cationic transfection reagents (e.g., charge ratio) the degree of purity of the transfecting nucleic acid (some cell lines are particularly sensitive to contaminants found in DNA preparations, such as endotoxins, salt and ethanol), the transfection time, the presence or absence of serum, the selection of a suitable reporter gene, and the amount of cells per reaction well, the medium pH (in calcium phosphate-mediated transfection, the required pH is 7.05-7.1). Further factors include cell health, confluency and number of passages.

Developing an approach which provides a satisfying endocytotic uptake efficacy is an art per se, and in many cases the respective conditions have to be newly adjusted in every new experiment. However, very often it turns out that in a transfection experiment, for example, a given cell line, which has turned out to be a useful expression host for many different proteins can not sufficiently be transfected with a given nucleic acid, even after extensive experimental testing.

With conventional methods, transfection efficacy (also termed "transfection rate", or "transfection efficiency") lies in the range of 0.0 % (in the worst case) and 60 % (optimum efficacy as advertised by respective Kit suppliers. However, as noted above, a satisfying transfection rate can only be obtained after extensive experimental testing.

Many kit suppliers, like Promega or Qiagen, provide troubleshooting guidelines which describe the above mentioned factors, and how to optimize them in order to enhance transfection and/or infection efficacy. It is however still an empirical process to optimize the respective conditions, and thus labor- and time consuming, in order to obtain a satisfying transfection efficacy. A systematic approach which reproducibly increases endocytotic uptake efficacy over a broad range of cells is still lacking.

It is thus the object of the present invention to provide a method for increasing endocytotic uptake efficacy of a cell. It is another object of the present invention to provide a cell, a cell line or an organism with increased endocytotic uptake efficacy, e.g., increased competence for transfection, or increased susceptibility for infection. It is yet another object of the present invention to provide agents or kits which allow the production of such cell, cell line or organism, or agents or kits which increase endocytotic uptake efficacy of a cell.

These objects are met with methods and means according to the independent claims of the present invention. The dependent claims are related to preferred embodiments. It is yet to be understood that value ranges delimited by numerical values are to be understood to include the said delimiting values.

### Summary of the invention

Before the invention is described in detail, it is to be understood that this invention is not limited to the particular component parts of the devices described or process steps of the methods described as such devices and methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. It is moreover to be understood that, in case parameter ranges are given which are delimited by numeric values, the ranges are deemed to include these limitation values.

According to the invention, a method for increasing endocytotic uptake efficacy of a cell is provided, which method comprises a step in which cellular activity of the Rab37 gene product, or an ortholog thereof, is impaired, or inhibited.

As used herein, the term "Rab37 gene product, or an ortholog thereof' relates, primarily, to the mRNA or the translated protein encoded by the Rab37 gene, as well as to its orthologues, which are described herein.

As used herein, the term "ortholog" refers to the relationship between proteins that have a common evolutionary origin and differ because they originate from different species or strains.

Endocytosis is the process by which cells absorb molecules (such as proteins) by engulfing them. Emdocytosis is a general principle for the uptake of compounds because most substances important for cells are large polar molecules that cannot pass through the hydrophobic cell membrane. Endocytosis pathways can be subdivided into four categories, namely, clathrin-mediated endocytosis, caveolae, macropinocytosis, and phagocytosis.

Clathrin-mediated endocytosis is mediated by small (approx. 100 nm in diameter) vesicles that have a morphologically characteristic crystalline coat made up of a complex of proteins that mainly associated with the cytosolic protein clathrin. Clathrin-coated vesicles (CCVs) are found in virtually all cells and from domains of the plasma membrane termed clathrin-coated pits. Coated pits can concentrate large extracellular molecules that have different receptors responsible for the receptor-mediated endocytosis of ligands, e.g. low density lipoprotein, transferrin, growth factors, antibodies and many others.

Caveolae are the most common reported non-clathrin coated plasma membrane buds, which exist on the surface of many, but not all cell types. They consist of the cholesterol-binding protein caveolin (Vip21) with a bilayer enriched in cholesterol and glycolipids. Caveolae are small (approx. 50 nm in diameter) flask-shape pits in the membrane that resemble the shape of a cave (hence the name caveolae). They can constitute up to a third of the plasma membrane area of the cells of some tissues, being especially abundant in smooth muscle, type I pneumocytes, fibroblasts, adipocytes, and endothelial cells. Uptake of extracellular molecules is also believed to be specifically mediated via receptors in caveolae.

Macropinocytosis, which usually occurs from highly ruffled regions of the plasma membrane, is the invagination of the cell membrane to form a pocket, which then pinches off into the cell to form a vesicle (0.5-5 µm in diameter) filled with large volume of extracellular fluid and molecules within it (equivalent to ~100 CCVs). The filling of the pocket occurs in a nonspecific manner. The vesicle then travels into the cytosol and fuses with other vesicles such as endosomes and lysosomes.

Phagocytosis is the process by which cells bind and internalize particulate matter larger than around 0.75 µm in diameter, such as small-sized dust particles, cell debris, micro-organisms and even apoptotic cells, which only occurs in specialized cells. These processes involve the uptake of larger membrane areas than clathrin-mediated endocytosis and caveolae pathway.

More recent experiments have suggested that these morphological descriptions of endocytic events may be inadequate, and a more appropriate method of classification may be based upon the clathrin-dependence of particular pathways, with multiple subtypes of clathrin-dependent and clathrin-independent endocytosis.

As used herein, the term "endocytotic uptake efficacy" relates to the efficacy of endocytotic uptake of a given agent, e.g., in an endocytosis experiment.

Endocytotic uptake efficacy is a prerequisite of competence of the respective cells, i.e., the ability of a cell to take up heterologous nucleic acids from the environment. Competence is distinguished into "natural competence", a genetically specified ability of bacteria that is thought to occur under natural conditions as well as in the laboratory, and "induced or artificial competence", arising when cells in laboratory cultures are treated to make them susceptible to heterologous nucleic acids.

Ras-related protein Rab-37 is a protein that in humans is encoded by the RAB37 gene. Rab proteins are low molecular mass GTPases that are known to play a role in cell vesicle trafficking. The inventors of the present invention have surprisingly found that the Rab37 gene affects the endocytotic uptake efficacy of said cell. Experiments revealed, among others, that impairing or inhibiting cellular activity of the Rab37 gene product increases said competence significantly, thus increasing transfection and/or infection efficacy.

This new finding is extremely beneficial for different purposes. In biotechnology, the transfection rate of a cellular protein expression system can be increased, and thus protein expression, e.g., production of industrial enzymes, biopharmaceuticals, like enzymes, antibodies or antigens useful as vaccines, can be facilitated.

In live virus vaccine production, the infection rate of a cell culture with a virus can be increased, and thus the production of a live virus vaccine can be facilitated. Further, the development of transgenic cell lines can become more efficient, and thus the production of transgenic animals (either chimeric or non-chimeric), or gene therapy applications can be facilitated. Other potential benefits of the present invention will be discussed elsewhere herein.

In a preferred embodiment of the present invention, said endocytotic uptake is related to transfection and/or infection of a cell.

As used herein, the term "transfection of a cell" relates to a process of deliberately introducing nucleic acids into a cell. In the present context, the meaning of the term is not restricted to certain cell types or certain vectors. The term "transfection" is therefore used synonymously with the term "transformation", which according to common understanding relates to the induced genetic alteration of a cell resulting from the uptake, incorporation and expression of exogenous genetic material that is taken up through the cell membrane. Likewise, the term "transfection" is used synonymously with the term "transduction", which according to common understanding is often used to describe virus-mediated DNA transfer. As used herein, the term "infection of a cell" relates to the infection of a cell by a pathogen, e.g., a virus, a bacterium, a mycoplasm or a protozoan, which results in the introduction of genetic material from the pathogen into the cell. Most pathogens infect a cell after endocytotic uptake. This is also the case for most enveloped virus, i.e. virus, which carry a lipid bilayer membrane as outer coating. Recent investigations showed that these viruses infect a cell after endocytosis, just like uncoated virus, while previously it was thought that they infect a cell by direct fusion of the lipid bilayer membranes of host cell and virus.

In these embodiments, increasing the endocytotic uptake efficacy means that the transfection efficacy or the infection efficacy is increased. As used herein, the term "transfection efficacy", or "infection efficacy", relates to the degree of successfully transfected, or infected, cells in a transfection or infection experiment.

In another preferred embodiment of the present invention, said endocytotic uptake is related to the uptake of molecules or particles, like proteins, cytokines, chemokines, growth factors, nucleic acids (particularly siRNAs, cDNAs and plasmids), nanoparticles, ligands, small molecular drugs or biopharmaceutical drugs. This approach can either be useful in order to affect cells in culture, or to affect cells in an organism, e.g., for gene therapy or cellular therapy. The uptake of siRNA, for example, can be used to inhibit the gene expression of a givene gene. The uptake of a cytokine or a ligand can trigger a given physiological response in the cell. The uptake of a cDNA can be used to create a cell which is transgenic for the gene encoded by said cDNA.

In another preferred embodiment of the present invention, said endocytosis-related transfection and/or infection is selected from the group consisting of
■ transfection with a viral vector
■ infection with a pathogen, e.g. a virus, a mycoplasm, a bacterium or a protozoan
■ liposomal transfection ("lipofection")
■ transfection mediated by calcium phosphate
■ nanoparticle-mediated transfection
■ TAT-PTD mediated transfection, and/or
■ cell-transfection with cationic polymers.

Viral vectors are a tool commonly used to deliver genetic material into cells. This process can be performed inside a living organism (in vivo) or in cell culture (in vitro). Viruses have evolved specialized molecular mechanisms to efficiently transport their genomes inside the cells they infect. Viral vectors are artificially modified versions of such viruses which are tailored to their specific applications, but generally share a few key properties. Although viral vectors are occasionally created from pathogenic viruses, they are modified in such a way as to minimize the risk of handling them. This usually involves the deletion of a part of the viral genome critical for viral replication. Such a virus can efficiently infect cells but, once the infection has taken place, cannot produce new virions because it lacks the respective proteins. Most viral vectors are engineered to infect as wide a range of cell types as possible. However, sometimes the opposite is preferred. The viral receptor can be modified to target the virus to a specific kind of cell.

Viral vectors often comprise certain genes that help identify which cells took up the viral genes. These genes are called markers, or reporter genes. A common marker is antibiotic resistance to a certain antibiotic. The cells having integrated the genetic material can be isolated as those that have not taken up the viral vector genes do not have antibiotic resistance and thus cannot grow in a culture with antibiotics present.

Non-limiting examples for viruses which have been used to create viral vectors are Adenovirus, Retrovirus, Lentivirus, Semliki Forest/Sindbis virus, Adeno-associated virus and/or Baculovirus.

Pseudovirions are synthetic viruses used to introduce genetic material, including DNA and RNA, with specific and desired traits into bacterial and eukaryotic cells. Pseudovirions are closely related to virus in structure and behavior but lack many characterists exhibited by true viruses, including the capability to replicate.

Many pathogens use endocytotic pathways for cell infection. A cell line, or an organism, in which cellular activity of the Rab37 gene product, or an ortholog thereof, is impaired, or inhibited, will demonstrate higher competence, or susceptiblity, to such pathogen infection. A respective cell line can thus be used for drug screening or infection research.

Further, infection of a cell culture, or a cell line, with a pathogen, e.g., a virus, can be used, e.g, for the production of vaccines. According to conventional live virus vaccine production strategies, hen eggs are infected with a virus, e.g., an influenza virus, which reproduces in the said eggs. The produced virions are harvested, processed (i.e., attenuated), and used as a live virus vaccine. The use of a cell line, or an organism, or even an egg, which has increased competence for viral infection approach due to impairment, or inhibition, of Rab37 cellular activity, can increase safety, speed and flexibility of virus vaccine production and reduce costs.

As used herein, the term "lipofection" (or liposomal transfection) relates to a technique used to introduce genetic material into a cell by means of liposomes, which are vesicles that can easily merge with the cell membrane since they are both made of a phospholipid bilayer, and thus makes use of endocytosis. Lipofection generally uses a positively charged (cationic) lipid to form an aggregate with the negatively charged (anionic) genetic material. A net positive charge on this aggregrate has been assumed to increase the effectiveness of transfection through the negatively charged phospholipid bilayer. The main advantages of lipofection are its high efficiency, its ability to transfect all types of nucleic acids in a wide range of cell types, its ease of use, reproducibility, and low toxicity. In addition, this method is suitable for all transfection applications (transient, stable, co-transfection, reverse, sequential or multiple transfections).

The person skilled in the art will understand that other endocytosis-related transfection methods described in the respective literature can be used in the context of the present invention, without applying particular inventive activity. One example for such alternative method is cell transfection mediated by calcium phosphate, which is a process not entirely understood. Calcium phosphate seems to precipitate the DNA which is to be transferred into the cell, and form a complex with the latter, which is then taken up by the cell by means of endocytosis.

Another example is nanoparticle-mediated transfection, e.g., ormosil-mediated transfection, in which the term "ormosil" stands for "organically modified silica". These particles, which are also known as nanoparticles, have turned out as highly efficient non-viral gene delivery vectors, while the uptake process is related to endocytosis. Other useful nanoparticles for this purpose are, e.g., thiolated chitosan nanoparticles, gold nanoparticles, or magnetic nanoparticles.

Another example is TAT-PTD mediated transfection. TAT (HIV-1 trans-activator gene product) is an 86-amino acid transcription factor of the integrated HIV-1 genome. The minimal PTD (Protein transduction domain) of TAT has been identified as a 9 amino acid (aa) protein sequence (RKKRRQRRR). A broad spectrum of therapeutic proteins can be fused to a PTD, so that PTDs can efficiently ferry cargo molecules into cells. TAT-PTD mediated transfection provides several advantages over the standard DNA transfection method in securing intracellular protein expression

Another method is the use of cationic polymers, such as DEAE-dextran, polyethylenimine or cationic lipds. The negatively charged DNA binds to the polycation and the complex is taken up by the cell via endocytosis.

In yet another preferred embodiment of the present invention, said impairment, or inhibition, of cellular activity of the Rab37 gene product, or an ortholog thereof, is caused by at least one step selected from the group consisting of
■ gene silencing,
■ gene knock down,
■ gene knock out,
■ delivery of a dominant negative construct,
■ conditional gene knock out, and/or
■ gene alteration.
with respect to the Rab37 gene product, or an ortholog thereof, or with respect to at least one gene necessary for cellular activity of the Rab37 gene product, or an ortholog thereof.

As used herein, the term "gene necessary for cellular activity of the Rab37 gene product, or an ortholog thereof' relates to all genes, which are involved in the Rab37 expression pathway, i.e., activity of which is necessary for cellular activity of the Rab37 gene product, or an ortholog thereof. This can for example apply to the Rab37 gene itself, as well as to genes involved in the regulation of Rab 37 gene expression.

Gene silencing or gene knock down refers to techniques by which the expression of a gene is reduced, either through genetic modification or by treatment with an oligonucleotide with a sequence complementary to either an mRNA transcript or a gene. If genetic modification of DNA is done, the result is a knockdown organism. If the change in gene expression is caused by an oligonucleotide binding to an mRNA or temporarily binding to a gene, this results in a temporary change in gene expression without modification of the chromosomal DNA and is referred to as a transient knockdown.

In a transient knockdown, which is also encompassed by the above term, the binding of this oligonucleotide to the active gene or its transcripts causes decreased expression through blocking of transcription (in the case of gene-binding), degradation of the mRNA transcript (e.g. by small interfering RNA (siRNA) or RNase-H dependent antisense) or blocking either mRNA translation, pre-mRNA splicing sites or nuclease cleavage sites used for maturation of other functional RNAs such as miRNA (e.g. by Morpholino oligos or other RNase-H independent antisense). Other approaches involve the use of shRNA (small hairpin RNA, which is a sequence of RNA that makes a tight hairpin turn that can be used to silence gene expression via RNA interference), esiRNA (Endoribonuclease-prepared siRNAs, which are a mixture of siRNA oligos resulting from cleavage of long double-stranded RNA (dsRNA) with an endoribonuclease), or the activation of the RNA-induced silencing complex (RISC).

Other approaches to carry out gene silencing or knock down are known to the skilled person from the respective literature, and their application in the context of the present invention is considered as routine.

Gene knock out refers to techniques by which the expression of a gene is fully blocked, i.e. the respective gene is inoperative, or even removed. Methodological approaches to achieve this goal are manifold, and known to the skilled person. Examples are the production of a mutant, which is dominatly negative for Rab37, or its ortholog. Such mutant can be produced by site directed mutagenesis (e.g., deletion, partial deletion, insertion or nucleic acid substitution), by use of suitable transposons, or by other approaches which known to the skilled person from the respective literature, the application of which in the context of the present invention is thus considered as routine. One example for a newly developed technique which the skilled person would consider as useful in the context of the present invention is knock out by use of targeted Zinc Finger Nucleases. A respective Kit is provided by Sigma Aldrich as "CompoZR knockout ZFN"

The delivery of a dominant negative construct involves the introduction of a sequence encoding for a dysfunctional Rab37 protein, or its ortholog, e.g., by transfection. Said encoding sequence is functionally coupled to a strong promoter, in such way that the gene expression of the dysfunctional Rab37 protein, or its ortholog, overrules the natural expression of the wildtype Rab37, or its ortholog, which, in turn, leads to an effective physiological defect of Rab 37 activity, or activity of its ortholog.

A conditional gene knock out allows gene to block gene expression in a tissue- or time-specific manner. This is done, for example, by introducing short sequences called 1oxP sites around the gene of interest. Again, other approaches are known to the skilled person from the respective literature, and their application in the context of the present invention is considered as routine.

One other approach is gene alteration, which may lead to a dysfunctional gene product, or to a gene product with reduced activity. This approach involves the introduction of frameshift mutations, nonsense mutations (i.e., introduction of a premature stop codon) or mutations which lead to an amino acid substitution which renders the whole gene product dysfunctional, or causing a reduced activity. Such gene alteration can for example be produced by mutagenesis (e.g., deletion, partial deletion, insertion or nucleic acid substitution), either unspecific mutagenesis or site directed mutagenesis.

According to another preferred embodiment of the present invention, said impairment, or inhibition, is caused by adminstration of, or incubation with, an inhibitor to the Rab37 gene product, or an ortholog thereof, prior to, simultaneously with, or after, transfection and/or infection.

Said inhibitor is, preferably, an agent interfering with the activity of the Rab37 gene product, by, for example inhibiting its GTPase activity, inhibiting the binding of a ligand to the Rab37 gene product, or inhibiting binding of the Rab37 gene product to a receptor. Examples for such inhibitors include, but are not limited to, an inhibitory peptide, an antibody, an aptamer, a fusion protein or an antibody mimetic against the Rab37 gene product, or a ligand or receptor thereof, or an inhibitory peptide or nucleic acid, or a small molecule with similar binding activity.

The term "antibody", as used herein, shall relate to immunoglobulins, or fragments or derivatives thereof. Particularly preferred, such antibody is selected from the group consisting of IgG, IgD, IgE, IgA and/or IgM, or a fragment or derivative thereof As used herein, the term "fragment" shall refer to fragments of such antibody retaining, in some cases, target binding capacities, e.g.
- a CDR (complementarity determining region)
- a hypervariable region,
- a variable domain (Fv)
- an IgG heavy chain (consisting ofVH, CH1, hinge, CH2 and CH3 regions)
- an IgG light chain (consisting ofVL and CL regions), and/or
- a Fab and/or F(ab)₂

As used herein, the term "derivative" shall refer to protein constructs being structurally different from, but still having some structural relationship to, the common antibody concept, e.g., scFv, as well as bi-, tri- or higher specific antibody constructs, antibody-based fusion proteins, antibody-drug conjugates, immunotoxins and the like.

The term "antibody mimetic" relates to a non-immunoglobulin-based target-binding protein molecule. Such antibody mimetics are for example derived from Ankyrin Repeat Proteins, C-Type Lectins, A-domain proteins of Staphylococcus aureus, Transferrins, Lipocalins, Fibronectins, Kunitz domain protease inhibitors, Ubiquitin, Cysteine knots or knottins, thioredoxin A, and so forth, and are known to the skilled person in the art from the respective literature.

The term "fusion protein" comprises multimers of the above-mentioned proteins plus a multimerisation domain, such as a leucine zipper or a coiled coil, or an Fc portion of an antibody, or a substantially similar protein, which is fused to a Rab37 binding domain.

The term "aptamer", as used herein, relates to an oligonucleic acid or peptide molecule that binds to a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications.

The present invention further provides a cell for use in protein expression, said cell being produced with one of the above defined methods, and/or having a modification leading to an impaired and/or inhibited expression of at least one gene necessary for cellular activity of the Rab37 gene product, or an ortholog thereof, and/or to the expression of a dysfunctional Rab37 protein.

In a particularly preferred embodiment, the impaired, or inhibited expression of at least one gene necessary for cellular activity of the Rab37 gene product, or an ortholog thereof, and/or to the expression of a dysfunctional Rab37 protein increases the yield of successfully transfected and/or infected cells in a cell transfection and/or infection experiment.

The cell according to the invention is preferably a mammalian cell. According to another preferred embodiment, said cell is an immortalized cell.

The cell according to the invention is preferably at least one selected from the group consisting of:
- HeLa cells
- African Green Monkey cells (Vero)
- Human Keratinocyte cells (e.g., HaCaT)
- Baby hamster Kindney cells (e.g., BHK or HKCC)
- Chinese hamster ovary cells (CHO)
- Murine myeloma cells/Mouse myeloma cells
- Human embryonic kidney cells (HEK)
- Human-retina-derived cells (e.g., PER-C6)
- Amniocyte cells (e.g., CAP)
- Madin-Darby bovine kidney cells (MDBK)
- Madin-Darby canine kidney cells (MDCK), and/or
- Avian cell lines from duck retina or chicken embryo (AGE1.CR, AGE1.CR.pIX, or PBS-1).

In another preferred embodiment, the cell according to the invention is preferably at least one selected from the group consisting of:
- yeast
- fungus
- insect, and/or
- protozoan.

Yeasts are eukaryotic micro-organisms classified in the kingdom Fungi, members of which have been used frequently in food production and biotechnology, particularly as expression systems. Preferred species are Saccharomyces cerevisiae, Schizosaccharomyces pombe, Pichia sp., and Hansenula sp.

As shown below, the genomes of yeasts comprise orthologs to Rab37, which in Saccharomyces cerevisiae are GTP-binding protein SAS1, YFL005Wp-like protein and Sec4p, and in Schizosaccharomyces pombe are GTPase Ypt2. These genes perform similar functions in the respective cells. Effects demonstrated herein for cells in which cellular activity of the Rab37 gene product has been impaired, or inhibited, can thus be transferred to yeasts in which cellular activity of one of the aformentioned gene products has been impaired, or inhibited.

Other fungi which find use as expression hosts are, among others, Hypocrea sp., and Trichoderma sp. The genetic equipment of these species is similar to the one of yeasts. For this reason, the above findings can be transferred to fungal expression systems, too.

Insect cells can be transfected with baculovirus based transfection systems, and are thus a popular tool for the production of recombinant proteins. Baculovirus-produced proteins are currently subject of many research projects. One insect ortholog of Rab37 is Rab26-RC, which is for example described for the fruitfly Drosophila, and seems to have similar tasks in the latter as Rab37 in humans. Effects demonstrated herein for cells in which cellular activity of the Rab37 gene product has been impaired, or inhibited, can thus be transferred to insect cells which are being used as expression systems, too.

Further, the present invention provides a cell line comprising cells according to the above defibitions. Preferably, such cell line is a human cell line or a mouse cell line. Other preferred alternatives are rat cell lines, hamster cell lines, canine cell lines, bovine cell lines or avian cell lines. Other preferred cell lines are insect cell lines, yeast cell lines, fungal cell lines or even bacterial cell lines, in which Rab37 or an ortholog thereof is involved in endocytosis.

Further, the present invention provides an organism comprising at least one cell according to any of the aforementioned claims. Said organism can either be fully transgenic, i.e., has been derived from a single cell produced with one of the methods described above. Alternatively, said organism can be chimeric, which means that the organism comprises cells from two different sources, e.g., one unmodified cell or cell line, and one cell or cell line produced with one of the methods described above. Such "knock out-", or "knock down"-organism can be used as in vivo model for screening or research purposes. Other potential uses of such organism are disclosed elsewhere herein.

The present invention is further related to the use of a cell, of a cell line and/or of an organism according to the above definitions as a host for heterologous protein expression, particularly for the production of a biopharmaceutical.

In this embodiment, the increased competence of the cell, cell line or organism is used to increase efficacy of transfection of the latter. The transfected cell, cell line of organism can then be used to heterologous protein expression, e.g. for production of industrial enzymes, like enzymes for feed technology, enzymes for the production of biofuels, enzymes for use in detergents, and so forth. Alternatively, such cell, cell line of organism can be used for the production of a biopharmaceutical, e.g., a protein therapeutic, or a protein vaccine.

The present invention is further related to the use of a cell, of a cell line and/or of an organism according to the above definitions, as a host for the production of virus or viral particles. In this embodiment, the increased competence of the cell, cell line of organism is used to increase efficacy of the initial virus infection of the latter, which is a bottleneck in current cell culture based virus vaccine production methods. Successfully infected cells, cell lines or organism can be used to produce virus or viral particles, which can for example be processed and be used as virus vaccine. This approach is as an alternative to conventional methods based on the infection of hen eggs; however, it suffers from poor initial infection efficacy. An increase of the latter, as made possible by the subject matter of the present invention, would increase the virus replication, and thus the yield of virus particles, i.e., vaccine.

The present invention is further related to the use of a cell, of a cell line and/or of an organism according to the above definitions, or of a method according to the above definition, as a tool for screening of candidate agents, drugs or vaccines which are supposed to affect at least one process selected from the group consisting of:
- endocytosis,
- transfection of a cell by a vector
- infection of a cell by a pathogen ("permissivity")

Such approach allows the quick discovery of new drugs or vaccines, which may affect the infection of cells by a pathogen, e.g., a virus, or the uptake of pharmaceuticals by a cell. Further, such approach allows the discovery of agents, which can be used as a tool in molecular biology or biotechnology.

The present invention is further related to the use of a cell, of a cell line and/or of an organism according to the above definitions, or of a method according to the above definition, as a high permissivity tool for screening candidate drugs or vaccines which are supposed to have anti-infective activity anti-pathogenic activity.

In this approach, the high permissivity of the cell, cell line and/or organism due to impairment or inhibition of the cellular activity of the Rab37 gene product, or an ortholog thereof, comes into play. Transfection or infection rate is increased, and such system or method is thus a feasible tool for the investigation of candidate drugs or vaccines, which are supposed to have anti-infective activity anti-pathogenic activity.

The present invention is further related to the use of a cell, of a cell line and/or of an organism according to the above definitions, or of a method according to the above definition, for use in endocytosis research and/or infection research.

The present invention is further related to the use of a cell or of a cell line according to the above definitions for the manufacture of a composition for use in gene therapy. Gene therapy is the insertion, alteration, or removal of genes within an individual's cells and biological tissues to treat disease. The most common form of gene therapy involves the insertion of functional genes into an unspecified genomic location in order to replace a mutated gene

In the case of germ line gene therapy, germ cells, i.e., sperm or eggs, are modified by the introduction of functional genes, which are ordinarily integrated into their genomes. Therefore, the change due to therapy would be heritable and would be passed on to later generations. This new approach, theoretically, should be highly effective in counteracting genetic disorders and hereditary diseases. However, many jurisdictions prohibit this for application in human beings, at least for the present, for a variety of technical and ethical reasons. In the case of somatic gene therapy, the therapeutic genes are transferred into the somatic cells of a patient. Any modifications and effects will be restricted to the individual patient only, and will not be inherited by the patient's offspring or later generations.

It is noteworthy that for transfection of the modified cells, endocytosis-transfection methods as described above can be used, i.e., transfection with a viral vector, liposomal transfection ("lipofection"), transfection mediated by calcium phosphate, or ormosil-mediated transfection. In this context, the method according to the present invention is highly beneficial, as it increases transfection efficacy and thus enhances the success rate of transfection.

The present invention is further related to a transposon, an antisense-RNA, an siRNA molecule, a pharmaceutical drug, an inhibitory compound or a peptide for the production of a cell according to any of the above definition. Such agent may be used for the impairment, or inhibition, of cellular activity of the Rab37 gene product, or an ortholog thereof, e.g. by gene silencing, gene knock down, gene knock out, conditional gene knock out, and/or gene alteration. Examples for siRNA molecules which are claimed according to the invention are given under SEQ ID Nos 6 - 9.

The present invention is further related to an inhibitor to the Rab37 gene product, or an ortholog thereof, said inhibitor being suitable for delivery, administration or incubation with a cell, which is to be transfected, or infected. Said delivery, administration or incubation may take place prior to, simultaneously with, or after, said transfection and/or infection. Examples for such inhibitor have already been described above.

Further, the present invemtion is related to a kit of parts, said kit comprising at least one inhibitor according to the above definition and/or a transposon, an antisense-RNA, an siRNA molecule, a pharmaceutical drug, an inhibitory compound or a peptide for the production of a cell according to any of the above definition.

In the following table, three different steps, which play a role with respect to the present invention, are shown, and examples are given for each of these steps. This listing is by no means exhaustive, or restricting, and the skilled person understands that other combinations, and other examples, may be used in this context, too, and still fall under the scope of the present invention.

Please note that the different examples for step 1 (impairment, or inhibition of cellular activity of the Rab37 gene product, or its ortholog), 2 (endocytotic uptake increased by step 1) and 3 (use of the cell thus obtained) can be permutated (e.g., example 1 for step 1 can be combined with example 3 of step 2, and so forth. Further note that these examples do not only apply for Rab37 itself, but for orthologs thereof as well as for genes which are necessary for cellular activity of the Rab37 gene product, or an ortholog thereof.

**Table 1**

| **Example No** | **Step 1** | **Step 2** | **Step 3** |
|---|---|---|---|
| | (impairment, or inhibition of cellular activity of the Rab37 gene product, or its ortholog) | (endocytotic uptake increased by step 1) | (use of the cell thus obtained) |
| 1 | Rab37 gene silencing, knock down, knock out or conditional knock out | Transfection of the cell with a viral vector | Production of a heterologous protein |
| 2 | Inhibition of Rab37 gene product | Liposomal transfection with a gene encoding a viral antigen | Production of a virus vaccine |
| 3 | gene alteration leading to dysfunctional Rab 37 gene product | Infection of the cell with a virus | Use of the cell line as research tool or for drug screening. |
| 4 | Rab37 gene silencing | Infection of a cell line with a virus | Use of the cell line for the production of live vaccine (e.g., attenuated influenza virus) |
| 4 | Rab37 gene silencing | uptake of a molecule or particle, e.g., siRNA | transitional gene silencing in a given cell |

### Role and definition of Rab37

Ras-related protein Rab-37 is a protein that in humans is encoded by the RAB37 gene. Rab proteins are low molecular mass GTPases that are known to play a role in cell vesicle trafficking. Rab-37 is a member of the human RAS oncogene protein family, and is encoded by the RAB37 gene. Gene details are as follows:
RAB37 [*Homo sapiens*]
Official Symbol RAB37 and Name: RAB37, member RAS oncogene family [*Homo sapiens*] Other Aliases: FLJ30284, FLJ32507
Other Designations: OTTHUMP00000165791; OTTHUMP00000165794; RAB37, member of RAS oncogene family; Rab37-like; ras-related protein Rab-37
Synonyms: B230331O03Rik, B230354I04Rik, FLJ30284, FLJ32507, MGC157622, RAB37 Chromosome: 17; Location: 17q25.1
Annotation: Chromosome 17, Inc_000017.10 (72667256..72743474)
Gene ID: 326624

The gemonic sequence of the human RAB37 gene is shown as SEQ ID No 5.

### Orthologs to RAB37

The following list gives an overview of orthologs to RAB37 in other species
- Mouse:: Official Symbol Rab37 and Name: RAB37, member of RAS oncogene family [*Mus musculus*]
Other Aliases: RP23-342L24.2, B230331003Rik, B230354I04Rik, MGC157622
Other Designations: GTPase Rab37; OTTMUSP00000003775; ras-related protein Rab-37
Chromosome: 11; Location: 11
Annotation: Chromosome 11,NC_000077.5 (114952745..115023554) Gene ID: 58222
- Rat:: Official Symbol Rab37 and Name: RAB37, member of RAS oncogene family [*Rattus norvegicus*]
Chromosome: 10; Location: 10q32.2 Gene ID: 363704
- Frog *(X laevis):*: Official Symbol rab37 and Name: RAB37, member RAS oncogene family [*Xenopus laevis*]
Gene ID: 100380956
- *C. elegans:*: rab-37, RAB family [*Caenorhabditis elegans*]
Other Aliases: W01H2.3
Other Designations: RAB family member (rab-37)
Chromosome: X
Annotation: Chromosome X, NC_003284.7 (4227833..4235822,
complement) Gene ID: 180696
- Guinea Pig:: ENSCPOG00000025645 (*Cavia porcellus*):
RAB37, member RAS oncogene family [Source:HGNC Symbol;Acc:30268]
- Rabbit:: ENSOCUG00000016341 (*Oryctolagus cuniculus*):
RAB37, member RAS oncogene family [Source:HGNC Symbol;Acc:30268]
- Fruitfly:: Rab26-RC (*Drosophila melanogaster*):
CG34410 gene product from transcript CG34410-RB
CG34410 [Source: FlyBase; acc: FBgn0086913]
- Yeast:: GTP-binding protein SAS1 [Saccharomyces cerevisiae RM11-1a];
Protein Accession: EDV09835.1
YFL005Wp-like protein [Saccharomyces cerevisiae AWRI1631]; Protein Accession: EDZ72422.1
Sec4p [Saccharomyces cerevisiae EC1118]; Protein Accession: CAY79446.1
- S. pombe:: GTPase Ypt2; Protein Accession: NP_594580.1

The following table gives an overview of Pairwise Alignment Scores of Human Rab37 in comparison with some orthologs

| **Gene** | | **Identity (%)** | |
|---|---|---|---|
| Species | Symbol | Protein | DNA |
| Pan troglodytes | RAB37 | 94.2 | 95.4 |
| Canis lupus familiaris | RAB37 | 91.4 | 89.6 |
| Bos Taurus | RAB37 | 87.6 | 83.1 |
| Mus musculus | Rab37 | 93.3 | 88.8 |
| Caenorhabditis elegans | rab-37 | 57.4 | 58.0 |

### Figures and Experiments

Additional details, features, characteristics and advantages of the object of the invention are disclosed in the subclaims, and the following description of the respective figures and examples, which, in an exemplary fashion, show preferred embodiments of the present invention. However, these drawings should by no means be understood as to limit the scope of the invention.
- Fig. 1:: Transfection efficacy enhancement by knockdown of rab37 with different siRNAs in HeLa cells
- Fig. 2:: Transfection efficacy enhancement by rab37 knockdown in HeLa cells
- Fig. 3:: Transfection efficacy enhancement by rab37 knockdown in Hek293 cells
- Fig. 4:: post- transfectional Rab37 knockdown has no influence on the protein expression activity
- Fig. 5:: Infection efficacy enhancement of infection by rab37 knockdown in HeLa cells.
- Fig. 6:: Infection efficacy enhancement by rab37 knockdown in Hek293 cells.
- Fig. 7:: Infection efficacy enhancement by rab37 knockdown in HaCaT cells.

### Experimental Procedures

### Cell lines and pseudovirions

The human cervix carcinoma cell line HeLa, the human embryonic kidney cell line Hek293 and HaCaT cells (human non-virally transformed keratinocytes), were grown at 37°C in DMEM supplemented with 10% FCS, 1% Glutamax I, 1% modified Eagle medium nonessential amino acids and antibiotics. HPV16 pseudovirions were prepared as previously described (Buck et al., 2004; Buck et al., 2005). Briefly, expression plasmids carrying codon-optimized HPV16 L1 and L2 cDNA (Leder et al., 2001) were cotransfected with a reporter plasmid coding for luciferase (Schneider et al., 2010) into 293TT cells. 48 hours post-transfection, the cells were processed to lysis and nuclease digestion. The pseudovirions were purified from the cell lysates by Optiprep gradient centrifugation (Buck et al., 2004). Pseudovirus yield was determined by luciferase-specific quantitative real time polymerase chain reaction (qRT-PCR).

### siRNAs

Four different siRNAs for targeting the human Rab37 gene have been produced according to standard methods. The target sequences were as follows:
Rab37-1: AAGGCGGATATGAGCAGCGAA (SEQ ID No 1)
Rab37-2: CTGTATGACATCACCAACAAA (SEQ ID No 2)
Rab37-3: CCGAAGCGTCACCCATGCTTA (SEQ ID No 3)
Rab37-4: CCGAGACTATGTAGAGTCCCA (SEQ ID No 4)

The siRNA sense sequence of the respective siRNA molecules was as follows:
#1: GGCGGAUAUGAGCAGCGAAtt (SEQ ID No 6)
#2: GUAUGACAUCACCAACAAAtt (SEQ ID No 7)
#3: GAAGCGUCACCCAUGCUUAtt (SEQ ID No 8)
#4: GAGACUAUGUAGAGUCCCAtt (SEQ ID No 9)

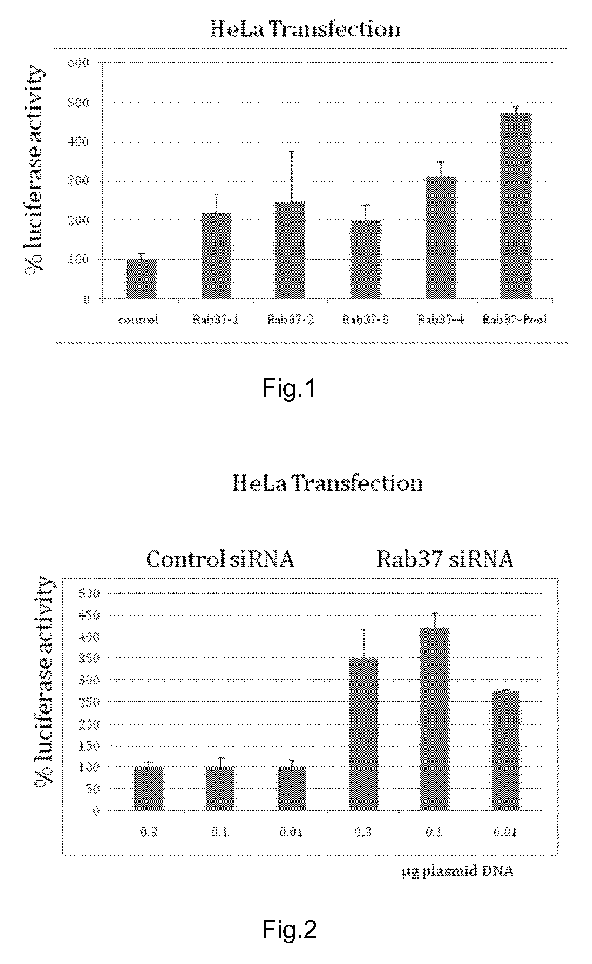

As non-silencing control siRNA, AllStars Negative Control siRNA from Qiagen was used. In preliminary experiments, siRNA-treatment with a combination of all four Rab37-siRNAs ("Rab37-pool") had the strongest effect on transfection efficacy of the luciferase reporter plasmid (see Fig. 1) and to reduce off-target effects, we used the Rab37 pool-siRNA for all further experiments.

### siRNA - Transfection Assays

Cells were seeded (25,000/well) in 24-well plates. The siRNA-transfections were done with Lipofectamine RNAiMAX (Invitrogen) and 30 nM siRNA according to manufacturer's recommendations. 24 h post-transfection, cells were transfected with a luciferase reportergene vector for 24 h. Transfection efficacy was defined by measuring luciferase activity using the Promega Luciferase Assay System. Cells were lysed in 250 µl Reporter Lysis Buffer and reporter quantitation was done in a multiwell plate luminometer with auto-injector using 180 µl cell extract and 50 µl Luciferase Assay reagent.

In a first round of experiments, HeLa cells were transfected with single and pool siRNAs. 24 h post-siRNA treatment, cells were transfected with a luciferase reportergene vector for 24 h. Transfection efficacy was defined by measuring luciferase activity. Fig. 1 shows that all knock down cell lines had an enhanced transfection efficacy (2 - 4,5 times) compared to the control.

In a second round of experiments, HeLa cells were transfected with Rab37 pool siRNA. 24 h after siRNA treatment, cells were transfected with different concentrations of a luciferase reporter gene vector for 24 h. Transfection efficacy was defined by measuring luciferase activity. Fig. 2 shows that all knock down cell lines had an enhanced transfection efficacy (2,7 - 4,2 times) compared to the control.

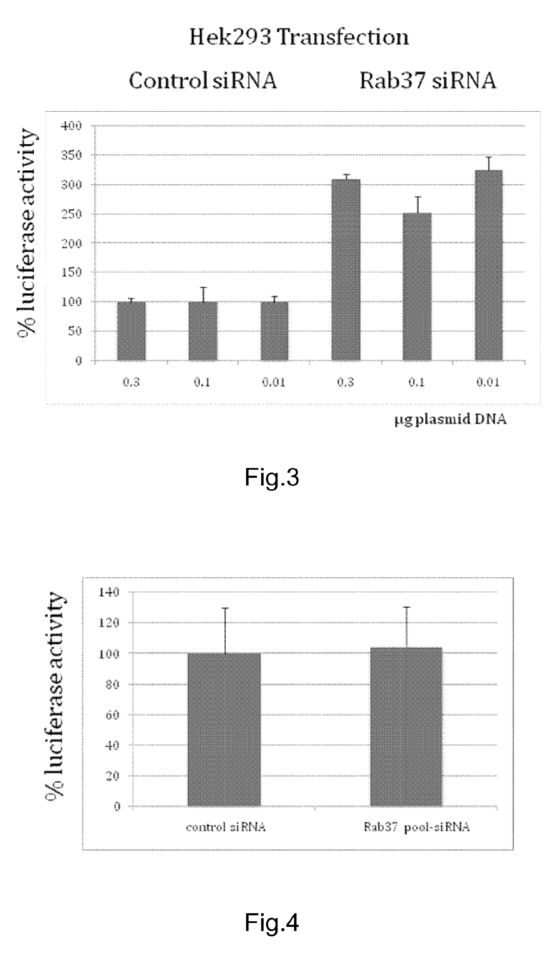

In a third round of experiments, Hek293 cells were transfected with Rab37 pool siRNA. 24 h post-siRNA treatment, cells were transfected with different concentrations of a luciferase reportergene vector for 24 h. Transfection efficacy was defined by measuring luciferase activity. Fig. 3 shows that all knock down cell lines had an enhanced transfection efficacy (2,5 - 3,3 times) compared to the control.

In a fourth round of experiments, which served to control the Rab37 knockdown on the expression-level of the luciferase gene, HeLa cells were first transfected with a luciferase reporter gene vector. 24 h post-transfection cells were transfected with Rab37 pool siRNA. Expression efficacy was defined by measuring luciferase activity 24 h after siRNA treatment. Fig.4 shows that the post-transfectional Rab37 knockdown had no influence on the protein expression activity.

### siRNA - Infection Assays

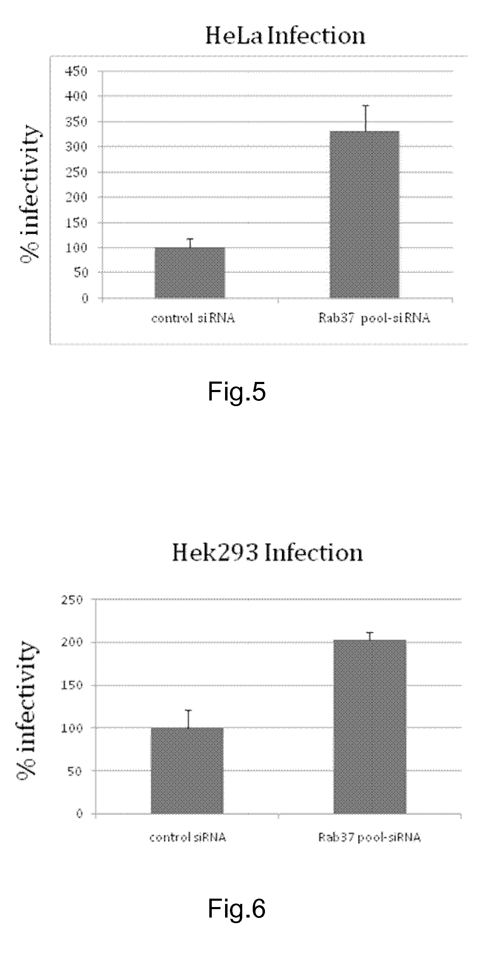

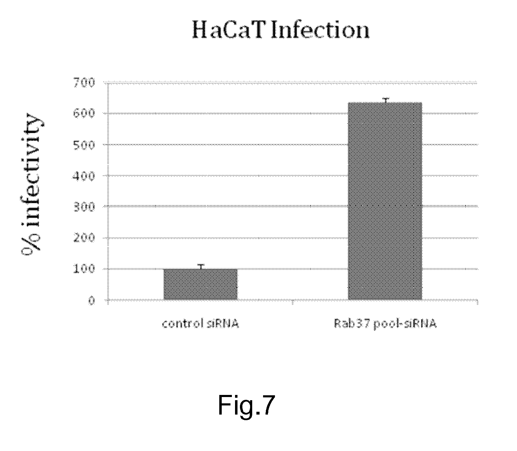

In a fifth round of experiments, cells (HeLa, HEK 293 or HaCaT) were seeded (25,000/well) in 24-well plates. Transfections were done with Lipofectamine RNAiMAX (Invitrogen) and 30 nM siRNA according to manufacturer's recommendations. 48 h post-transfection, cells were infected with HPV16 pseudovirions bearing a luciferase reportergene vector for 24 h. Infection efficacy was defined by measuring luciferase activity using the Promega Luciferase Assay System. Cells were lysed in 250 µl Reporter Lysis Buffer and reporter quantitation was done in a multiwell plate luminometer with auto-injector using 180 µl cell extract and 50 µl Luciferase Assay reagent. Figs. 5, 6 and 7 show that the respective knock down cell line (HeLa, HEK 293 or HaCaT) had an enhanced infection efficacy (2 - 6,3 times) compared to the control.

### References

Buck et al. (2004): Efficient Intracellular Assembly of Papillomaviral Vectors. Journal Of Virology, Vol. 78 (2), 751-757
Buck et al. (2005): Maturation ofPapillomavirus Capsids. Journal Of Virology, Vol. 79(5), 2839-2846
Leder et al. (2001): Enhancement of Capsid Gene Expression: Preparing the Human Papillomavirus Type 16 Major Structural Gene L1 for DNA Vaccination Purposes. Journal Of Virology, Vol 75(19), 9201-9209
Schneider et al. (2010): Identification of the dynein light chains required for human papillomavirus infection. Cellular Microbiology, doi:10.1111/j.1462-5822.2010.01515.x

## Claims

1. A method for increasing endocytotic uptake efficacy of a cell, which method comprises a step in which cellular activity of the Rab37 gene product, or an ortholog thereof, is impaired, or inhibited.

2. The method according to claim 1, wherein said endocytotic uptake is related to transfection and/or infection, and/or to the uptake of molecules or particles.

3. The method according to claim 2, wherein said transfection and/or infection is selected from the group consisting of
■ transfection with a viral vector
■ infection with a pathogen, e.g., a virus, a mycoplasm, a bacterium or a protozoan
■ liposomal transfection ("lipofection")
■ transfection mediated by calcium phosphate
■ nanoparticle-mediated transfection
■ TAT-PTD mediated transfection, and/or
■ cell transfection with cationic polymers.

4. The method according to any of the aformentioned claims, wherein said impairment, or inhibition of cellular activity of the Rab37 gene product, or an ortholog thereof, is caused by
■ at least one step selected from the group consisting of gene silencing, gene knock own, gene knock out, delivery of a dominant negative construct, conditional gene knock out, and/or gene alteration with respect to the Rab37 gene product, or an ortholog thereof, or with respect to at least one gene necessary for cellular activity of the the Rab37 gene product, or an ortholog thereof, and/or
■ adminstration of, or incubation with, an inhibitor to the Rab37 gene product, or an ortholog thereof, prior to, simultaneously with, or after, transfection and/or or infection of the cell.

5. A cell for use in protein expression, said cell
■ being produced with a method according to any of claims 1 - 4, and/or
■ having a modification leading to an impaired and/or inhibited expression of at least one gene necessary for cellular activity of the Rab37 gene product, or an ortholog thereof, and/or to the expression of a dysfunctional Rab37 protein.

6. The cell or method according to any of the aforementioned claims, wherein the impaired, or inhibited expression of at least one gene necessary for cellular activity of the Rab37 gene product, or an ortholog thereof, and/or to the expression of a dysfunctional Rab37 protein increases the yield of successfully transfected and/or infected cells in a cell transfection and/or infection experiment.

7. The cell according to any of claims 5 or 6, wherein said cell is a mammalian cell, preferably an immortalized cell.

8. The cell according to any of claims 5 - 7, wherein said cell is at least one selected from the group consisting of:
■ HeLa cells
■ African Green Monkey cells (Vero)
■ Human Keratinocyte cells (e.g., HaCaT)
■ Baby hamster Kindney cells (e.g., BHK or HKCC)
■ Chinese hamster ovary cells (CHO)
■ Murine myeloma cells/Mouse myeloma cells
■ Human embryonic kidney cells (HEK)
■ Human-retina-derived cells (e.g., PER-C6)
■ Amniocyte cells (e.g., CAP)
■ Madin-Darby bovine kidney cells (MDBK)
■ Madin-Darby canine kidney cells (MDCK), and/or
■ Avian cell lines from duck retina or chicken embryo (AGE1 .CR, AGE1 .CR.pIX, or PBS-1).

9. The cell according to any of claims 5 - 7, wherein said cell is at least one selected from the group consisting of:
■ Yeast
■ fungus
■ insect, and/or
■ protozoan.

10. A cell line or an organism comprising at least one cell according to any of claims 5 - 9.

11. Use of a cell according to claims 5 - 9, or of a cell line or organism according to claim 10 as
■ a host for heterologous protein expression, particularly for the production of a biopharmaceutical and/or
■ as a host for the production of virus or viral particles.

12. Use of a cell according to claims 5 - 9, or of a cell line or organism according to claim 10, or of a method according to claims 1 - 4,
■ as a tool for screening of candidate agents, drugs or vaccines which are supposed to affect at least one process selected from the group consisting of endocytosis, transfection of a cell by a vector, and/or infection of a cell by a pathogen ("permissivity")
■ as a high permissivity tool for screening candidate drugs or vaccines which are supposed to have anti-infective activity anti-pathogenic activity, and/or
■ in endocytosis research and/or infection research.

13. Use of a cell according to claims 5- 9 or of a cell line according to claim 10 for the manufacture of a composition for use in gene therapy.

14. A transposon, an antisense-RNA, an siRNA molecule, a pharmaceutical drug, an inhibitory compound or peptide for the production of a cell according to any of claims 6 - 11, of a cell line or of an organism according to claim 10.

15. An inhibitor to the Rab37 gene product, or an ortholog thereof, said inhibitor being suitable for delivery, administration incubation with a cell which is to be transfected, or infected, said delivery, administration or incubation taking place prior to, simultaneously with or after said transfection, and/or infection.


SEQUENCE LISTING

<110>  Universitätsmedizin der Johannes Gutenberg-Universität
       Mainz

<120>  Cell transfection under Rab37 deficiency or inhibition

<130>  UD40254

<160>  9

<170>  PatentIn version 3.5

<210>  1
<211>  21
<212>  DNA
<213>  artificial

<220>
<223>  Rab37 siRNA target sequence

<400>  1
aaggcggata tgagcagcga a                                                21


<210>  2
<211>  21
<212>  DNA
<213>  artificial

<220>
<223>  Rab37 siRNA target sequence

<400>  2
ctgtatgaca tcaccaacaa a                                                21


<210>  3
<211>  21
<212>  DNA
<213>  artificial

<220>
<223>  Rab37 siRNA target sequence

<400>  3
ccgaagcgtc acccatgctt a                                                21


<210>  4
<211>  21
<212>  DNA
<213>  artificial

<220>
<223>  Rab37 siRNA target sequence

<400>  4
ccgagactat gtagagtccc a                                                21


<210>  5
<211>  76219
<212>  DNA
<213>  human

<400>  5
agctgcgtgg tgacggagat gcccgttcgc tcgggatcag gaacggtgag ctcctgggac      60

cgcaatgccc tgagaacgca gccgggtgct gagcctccgg agtccggtag ctgaatgaaa 120
tacgcaggga tcaccagccg gacccaaatc ttgcgtccct gccagcatcc tggagtccta 180
agaggcaggg attggagcgg acaggatctc agaactctgg tcccgggcgc acaacggcgg 240
agtcgctgtt cctggtgctg aaacgctcag tcctggaaga acgtggccgc ctgcccgcct 300
ggtaccgcgc cgcggccgct gcggggaact gtccagtgct gaaaacggat gcggcccggc 360
ccgcagagct cagacccaag cctgccgcac ccagcggagc tcgaaccgag ctcctggaag 420
cgctgacgca gagcgcaggg agagccggag cggcgagctc caagcctggc atggacctgc 480
agagacccga ttcctaccag ggaggagctg gccctgactt caacgaccac gtcctgcata 540
aggtaaacat ctcctgtatt cctcaaccta acgttggaag aggcgccagc accaggagtt 600
ttctccactc ccctgacttt gctttgggac ttaatgagaa actagcttgt atctgtgagt 660
ctggggagcc ctttctgtct gatcctgttt cctgctcaaa acagagatgc gtgagctctt 720
ggggaagggc tgccgccaga ggctcagatg gtcagaagct tcattcattc acttacatca 780
tctgtgttcc tttttcaccc cctccttctc cttgcctatt tatgcccctg ggtgacccct 840
ggcaccttgc tctcctaacc cttgtaggag actgcatttc tccctgttac atctgctagc 900
agtggccaaa tacctgaatt tctggccaat ttgtctgaaa ttagataaat ggtctgcagc 960
agagatgaac tgctgttttc aaatcagact ccctcatggc tccagaaagc ttccctccag 1020
ccatgattcc ctcacagccc ctgtaagcat ttgcttattt caaagcacta acatatccat 1080
tgcttccttc actcaagttt cttatgcaac tcagttttca gtaatgcagg tctcccccta 1140
agatagttgt tgggaaaaat agtagcctct ctcaggataa cagttttaat tcaaaatcca 1200
tcactctgaa ataatcaaag tgttatattt gccataagaa gttattatgg ctcaccttcc 1260
ttttctctga ctttgccatg ccttcctgct ccagccccta tattttctgc tttgataatg 1320
ccagttcacc tgctagtcct ttgaaaagct gtacgtgatc ctataggtgg agttttttaa 1380
tcaaaacatc actttgaaga gtctatgaat gcaagctttg aaataaccta acccaacctc 1440
ctcctctatc acccagctta tatttgagga aactaaaatc cagagaggaa aaatgacttg 1500
tccaagaaag caagctggta aatggcagag ctgatactaa aagccagaaa ttactactac 1560
acgtgtagta ttctttcctc tatatcctta ctaaacttct catcaaagtg tgcagtccat 1620
ggaccagcag cattggcatt accttggaac ttattacaat agaaagccct gaccctagat 1680
tggctgaatc tgaatctgca ttttaacaag atctctagga ataaatatgc acaataaagt 1740
tttaggtgca tggctctgtg ccatgctgcc tgtttctgac acaaatgaaa gaaaatcagc 1800
tattgaagga agcaggtctc tagatctgac agtccatgtg tcttctccct ccaggtctct 1860
gaattcctag gatatagaga aggagaaaaa atgtataaca actatcccag tctagaaatt 1920
ttaaagccat cacagtttga agtaaactag aagaaattct gtgctatttg atgttgtcaa 1980
tttagcccag taaaaactgc atgcaaggcc aggcatggtg gctcacacct gtaatctcag 2040
cactttggga ggccgaggcg gtggaccgct tgaggtcaag ggttcaagac cagcctggcc 2100
aacatggtaa aatctgtctc tactaaaaat acaaaaattc gctgggcatg gtggtgtgcg 2160

cctgtgatcc cagctactca ggaggctgag gcaggagaat tgcttgagcc tgggaggcag 2220
agactgcagt gaaccgaaat cacaccactg cactccactc tagcctgggc aacaaagcaa 2280
gaccttgtct caaaaaaaaa aagaaaaaaa aagtttatgc aagcagagga acataattac 2340
aaatcaatta caaaattttc ctggcaacta gatatgtggg tctctccaag tttataccat 2400
cctataaagg tataaatact cattttgaaa gctttaagat tgggagctca gattgttcaa 2460
aatattgatc agatgtggta ttttccattt caattaccca cacacgagat gtaggatgta 2520
gtccatgaag actgttactt cctttgagtt tattgatcac caaattgaga gtgggaaccc 2580
attccaacaa gttccaacat atttaaaggc aagtatctat ttactactag taaaggctca 2640
atgtcatcag cttggctgtg tgagcaccct gagagcagga gccagcctta aaccttctgt 2700
ggtgcccagc agagtgacat atgtatgtaa ggaaagtttt ccttcttata tagtgctgac 2760
aacctcctcc taatactact ttatgattgt gcatgatgtt ttattgtgat ccacttaaaa 2820
ctctttttgt aaaggggtag ggcatcaact tatttgtatt atattaaaaa ttactatgta 2880
gtagagtcca aggagttccc attgattgat tagtgtctat tgtccatgag aagcagggct 2940
cccctccaac attaattgct aggataaatt ctttttttga agtggggtct cagtctgtca 3000
cccaggctgg aatgcagtgg tgtgatctca gctcactgca acctctgcct cccaggcaag 3060
tgattctccc acctcagcct ccccagtaac tggaactgca ggcatgtgcc accatgcctg 3120
gctaattttt tggcagttgt tgttttgtag agacagagtt tcaccatgtt ggccaggctg 3180
gtcttgaact cctggcctca agagatccac ccgctcggcc tcccaaagtg ctggcattat 3240
atgtatgagc caccatgccc agcgtgggga aatgattttt taaatagact tgttattgac 3300
acataacaaa caccactttg ggaagctgag gcgggcagat ctcttgaggt caggagttca 3360
agaccagcct ggccaacatg gcgaaacttt gtctctacta aaaatacaaa aatcagctag 3420
gcgtggtaac acactcctat aatctcagct actagggagg ctgagacagg agaatcactt 3480
gaaccgagga ggtggagact gcagtgagcc aagatcacac cactgcactc cagcttcagc 3540
aacagagcaa gactccatct caaaaaaaca aaaagaaata tgacaaacac caaaaaatcc 3600
tgaatcttat gtgtctacct tggtgaatta tcacaaagtg aacatacctg tgcaatcact 3660
atcaagagaa agaaatagaa ccttgtcagc ttcccaagag gccccctccc acattccact 3720
tgccaaacac cctcccccag agattgtcat tatagaggaa gaagcatcat cttgtgtttt 3780
gacagcacag cagatgttat gtagggtagt cctagaaact tgccagagaa gagatattgt 3840
cgtatgatga aacgaaactc caggcttgga ataagccaaa ccttgatgtg actttgagca 3900
acatctgtga gtcttaattt tcttagctat aaaataggaa tgataatacc tgccttatta 3960
tttttgagca aggatgttag cgaatataat gtaacgcgta tatataaaat ctctggaata 4020
taggaagcat tcaataaaag tggttgtatt ggctgggcgc agtggctccc agcactttgg 4080
gaggccaagg aggatgaatc atgaggtcag gagatcgaga ccatcctggc caacatggtg 4140
aaaccccatt tctactaaaa atacaaaaat tagccggggg tggtggcatg cacctgcagt 4200

cccagctact caggaggctg aggcaggaga atcgctttaa cctgggaggc agaggttgca 4260
gtgaaccaag atcacaccac tgcacttcag cctggtgaca gagtgagact ccatccgaaa 4320
aaaaaaaaaa agtggttgta ttagggcatg catcttgggc tccaaagcag cttcaatggg 4380
tgtcacattc agtctcacct aaaatgatga gctttgtgag aagagcagca gcttcataca 4440
agagaatctc agaaacacac acactaccta tttcacaggc agaagtccat ggaaataaac 4500
tcacacaacc catctataca aaaagcgtga cttctatgca aacatcccag ttctgacttc 4560
ctaagttgag tatctttttt ctcttggagg tggtgtatgg gacttgatga aattcattta 4620
tatgaaaaac atacgccaaa tatcatgata ggcattgtgt agctcttgca ctttagctgg 4680
tcccaatagt tggaggctga cccaggcaag cccaagtaac tcttagaaga ttcccgtagg 4740
gtgggctgga agctgggaac tgactgaaaa cattgagctc atccatcatg tgcctggagt 4800
ttttaaatag agttgttgtt gccacaaatc tgaaaatttg gtgcagctaa ataatttaaa 4860
acaaaggagc aacctggctg ctcttttcct tagagaactc tcagttctct cttgccacca 4920
gcttcaagag ccatccaggg caatggtctg gggtaggggt tcaggaacag cccacggatt 4980
gggtccctac tgtgccaggg tctgaccttg agctcccaga aaatgaaaag attttcattt 5040
cattttcaca gagagggcaa gcttttgccc cagataccag tgaattagga caaaagggct 5100
tcagttcaag cacttacttc ctaattccct atcaccaggg ctctaaggaa catccacata 5160
ccagactagc ttccagaggg acgaacaatt cctaggacaa agtcagtctc agaaagactg 5220
ggggtggggg gcagaaaaac gtgagctgct catccattca tccagggctt ggttcttctt 5280
gggtgaaaag ggattaccag gtctctgtct tccaaagtat gtccatttcc tccgactgct 5340
ctaacacctc ttctgggtga aaggaaaagg caagcaggta atatctgcca ctctccccca 5400
tctcctgcac ttattcttca cttccttctt ctattaccca tcttgtaatt tctgtgtccc 5460
tcaaaagtcc gcatgcctca tcgattcatt cagcaaacat gcattggaca tttccaaagc 5520
gtaaagaact aggctggtca ttgtcgagaa tatagacgca aatgattccc accttcacta 5580
agcctgtggc ctagaaggca tgcacacaaa tggctatact tttatataat tccagaagtg 5640
tcctaagaaa gatacgggta gctgcaaggg agtgacccag gaagaaaagg tcatgtctga 5700
ctgaatttga aatgtcctct ctcacttatg gcttctgatt aaagttaacc tccaggcttg 5760
gcacggtggc tcacacctgt aatcccagca ctttgggagg ctgaggcagg tggatcatga 5820
ggtcaggaat tcgagaccag cttggccaac atggtgaaac tcgtctctac taaaaataca 5880
aaaattagtc agccgtggtg gtgcacgcct gaaatcccag ctactcagga ggcagaggca 5940
ggagaatcgc ttgaacccag gaagcagaag ttgcagtgag ctgagatcac accattgcac 6000
tccagcctgg gtgacagagt gagactctgt ccccccacaa aaaaaattaa aattaaaaaa 6060
taaaaaataa agttaacatc catgagagac agagttccac acatcagcta gctccctccc 6120
catgggactg tcacctatta atcctaggga aaggtctgga tacaacagtg ttctccttcc 6180
ctgccagaaa attgtacaag agaaagacaa ataaaacagc aaaagaccat actcgccatt 6240
ctgtgttcct ctctcattct ctatcccaca ggtgcttctt ttattttttt ttcgatgaaa 6300

caatgataat gataatggta tgcaaatttg tcctcaaaag aatccaaatt agcagaaaaa 6360
tccatgccct gtaagtgaag agagttttgt tttcaaagcg cactagttcc aattctcaaa 6420
gatttctatt gtcagctcag gaacaaacaa gaaagccaag aaaagctagc tgctccctgt 6480
ataatatttc cactggcttg ttttcctctc ttgcttgctc tgaaggctta cagataaaca 6540
aatgaagtgt atattacatc actggcaatc tggtgccgtc tgggactcct ttggctgata 6600
ggtcggtcct gcccagatcc cagagcaggc agctggtgtg gggagtggtg gtgcctgcta 6660
gttctcttag gataacctca gcaggaaatg atggtgactc agtccagcgc ctcaaagtcg 6720
ttttccaaac tctccaggct ttcctctcct aagcatcctt tcctttaatt tgtggtccct 6780
ggggcctttt gtgagaaaat gtctgagact cacttgtgtt gctgccctaa tgtttcctct 6840
tataagctgt tctagggagg gacgtccttc agtttcaggg caggaaactc cgccttactg 6900
ggtggaagta aacagccaga ggtgagcacc aggctggaga attgttgaag tgggcatttg 6960
aagtggctct aaatttatcc caataccctt cttgcttcct tcctcctctt ctcaggcaag 7020
agaaacagtc tgtagagact gcgagtgcga atggagatca cagctccttg ttaactcact 7080
caggcaatgt agttcccagc tgggagtggc cctggacatc ctcctatctc ttaaccagag 7140
gaggttttct ggggccaaga gagacagctc caagcaaaca gatctggaaa atattaaccc 7200
ctaactgatg acaccaggcc tcaccctgct cctaaaggga gcaaggcctt gagtggtgca 7260
gtgaaaataa cttgagacgt gcagggctca agaattctcc cagcacatgg ttcctgacct 7320
tgatttctaa gtcagctccc tcccttctca tttgcttatc agcgcctttt catttgctta 7380
ttagcacctt ctcatttgct tattagcaga cagtttctac ccatcaacat gaggatgcta 7440
tttaatcatt tgtgcttttg caaatgcaca tgcttttagg tatctgaaac caagctgtgc 7500
ttaagttcaa attttctctt acttgctagc cttctgcccc tggccaccac acacacagac 7560
acacagacac acacacacac acacacacac atgcctgcac atgcacacat acgcaccctt 7620
cctctcatga gctgtgggca gggccagtga tcatccgttt ttgttctctg tctcccctta 7680
gcttttgtta tgagcctttt aaaagcaaag caggctgggt gcagtggctt agtcctgtaa 7740
tcccagcact ttgggagtcc aaggcaggca gatcacctga ggtcaggagt tcaagatcag 7800
cctggccagc atgaggaaac tctgtctcta gtaaaaatac aaaaattagc tgggcgtggt 7860
ggcaggcgcc tgtaacccca gctacttggg aggctgaggc aggagaatca cttgaacccg 7920
gaaggctgag gttgcagtga gccgagatcg caccactgca ctccagcctg ggcaacaaag 7980
caagactctg tctcaaaaaa aaaaaaaaag gcaaagtaaa catttctcct cctcttcttc 8040
ctccctcgcc tgtttgaatt ttgcattcat accctctccc agcaactctg tttattaaaa 8100
atcggaactg tcatttatta gagcagtacc cctaggggag agtcaggaac tggtaacacc 8160
ttccccaagc tcacagtgag aagcagtgta agctttggaa gggaataaag tctggtacac 8220
agtccagatg aggcagacac cccctttcag cccctggagt ctccctcctc gcctccccca 8280
tgcctttacc cagatccttc ctccttctct gggtcccacc tcaggtttca tttggttatc 8340

aggtaagaaa tattaaaaat catctctgca ctataatcca aagggaccca gtaagactaa 8400
taattgttcc caatacatta tccaaataag catatggttt atttctgcag cactttacag 8460
tttgcgaaac actttaatat atgccaagac attttatact taacatatgc tttttccaga 8520
agatgttact attaaactaa ttaatgtcca tattaatagg acagggaaac aaaggccaga 8580
tggctgggca tggtgcttca tgcctgtaat cccagcactt tgagaggctg agatgggcag 8640
atcatgaggt cagtagtttg agaccagcct ggccaacatg gtgaaaccct gtctctacta 8700
aaaacacaaa aattagctgg gtgtggtggc gggcacctgt aatcccagct actcaggagg 8760
ctgaggcagg agaattgctt gaacccagga ggcggaggtt gcagtgagct gagattgcga 8820
cactgcactc cagcctgggt gacacagcaa ggctctgtct caaaaaaaaa aaaaaaaaga 8880
aagaaagaaa gaaaaaactg acttgctcaa ggtcatagcc agcattgtgg aagcctgaat 8940
cattacagca gacccttaga tagcacttgc atgccatgta atattctaag cacttagcat 9000
ctcttaactc acttaatcct acaacaaacc tattttaatc tcattttaca gatgagtaaa 9060
ctgaggcatg gagacattaa gaaatttgcc tatagtcaaa tccaagcaag gtagacaggc 9120
agagggatag agtcaggaga ggacaagggg cttcaccaag agggtcacag atggtcacta 9180
ctttagggca ttgacagcag tccattgtgt ggccttcagc tctgtagagc tggttttgct 9240
cactcactca agagtggctc catctctcca gtcaggctga tgttaatcag actcacaaga 9300
catgtattgc cctagatgcc aagccacatt ataatcagga gatttttttg ttcgctaagc 9360
atgaaatcca ggggaaaaaa tggaacttgg aggattcaaa tgtttaatgc tatcaggctg 9420
acatgagttg gtagctggct acactatagg cacagcctca atctacgacc ccactggccc 9480
cttctaccct taggtcctct cccctacccc ttgccccacc tctgcctccc tcagctgtat 9540
atatttaact gccttgggcc ggacagtcat tctattctct ccaggtctgt atttcctgca 9600
atcctttaag aactccttgc ctttgagctt gagaattccc tggacttgac ctggttatgg 9660
ggagggaaag ggaagggatg ttccgttcat catcgccaga gttgtcattt catacttctt 9720
cctccatctc actcaaggcc tgtgtcagag ccttagggac ttggatttca ggggcaagaa 9780
ccatggaaga gtagaaaggt gattggggac atttccttat accccaccac cacccctgtt 9840
cacatgctcc ccttgaaatc tgatatttct gcctgagaat cctgagaatc aattcagcaa 9900
atatttactg cgcaccacat gccagtcccc aggaacaaaa cccactcttc attccccaag 9960
gagctgcgtc cagcaggctg ggtggatcag agggatggca caggcagaga tggctcaggc 10020
cacatctgac tgcagctcag tgtgccacgt ggaccttggt gcccatgaag gtcaaagacc 10080
tctagtccca tatagtggga agctgagccc ttggggacca ggtcaaaagc ttgcagctct 10140
cgaggctgcc aacttctggc tcctggctga ttgacggatc tgaggggtta taaagcccaa 10200
agtcccagga tggggcccca cctctgaagg tcagagggct ctgagcccct caacacaggg 10260
atcccttcaa gcccatgtcc aagaaaggaa gagactcctg ttgcacttct gagaagtgcc 10320
cagtgcaaag acccctgtcc aattgtggca gagtccaaca catagaaact aagctggatc 10380
ccttccatga ccatctccaa agacatgggc tgaaaaacac tcaaccaaac ccaaaacacc 10440

aggatcccag cccttgggat gacagtgtgg cacacaaaga gaggcttggg aaaacagagg 10500
ggcacgccac gtgagctctg caggcatgtc ctccaggacc ctcactctcc tttccctgaa 10560
accaggtctc ctccagacag caggcacatg agaaccagca tagcataggc cctgaccatc 10620
acctgtcctg accaccacct gtgctgtcta ccacctgtcc tgttcaccac tggttccatc 10680
caccacctgc cccatccatc acttgttctg tccaccacct gtcttgtcta ctgccttccc 10740
tcttcaccct catcctgttc actacctgcc ctgtccacca cctgtcctga ccaccttctg 10800
ccctgctcac tgattttctt agagtaggag gcagctcatt agcagagaaa gagaactggg 10860
ctttgagtac caaagaacca agctttatcc ctgtctctgc tgttagccag ctgtacattc 10920
taggtcattc cattccaaca gcactttcca acttgcagaa aacttgcata taaatcattt 10980
cggttgaatt ccccagcact gtgtgtaaag agaacaggca ttattcttga ctgcaaaagg 11040
ggaaatcttg tgatcaggac tctcgatttc cagtgacaaa acccaactca aattggctta 11100
agccgaaaag agaactcatt ggttcctgta ccagagaagc ctagggagtg cagctgacat 11160
cgagctcagc tggtcccagg gactctcagt gacttcggcc tcactctctg tctctctctc 11220
tctctcattt ttctctctgg cttggctcca ttcacaggca ggcttctccc cgtggggccc 11280
cattattgac agcagcccca ggatcacatc ctcacagctc ccaattccaa acaaagtcct 11340
agacccaata ctcactggag actgaaattg ccacatgccc acccctgaac caatactata 11400
gccagagggt agaacatgat ggacggctta agctcgggtc acatgtccac cccgaaggtg 11460
gagtcaatcc cacccaagcc ctgtagatca agagtggaag aaaatgggcc gggcgtggtg 11520
gatcatgctt gtaatcccag cactttggga ggtcaaggcg ggtggatcac ccgaggtcgg 11580
gagttcaaga acagcctgac caacatgggg aaaccccttc tctgctaaaa atacaaaatt 11640
agccggttgt ggtggtgcat gcctgcaatc ccagctactc aggaagctga ggcaggagaa 11700
tcacttgaac ccgggaggcg gaggttgcgg tgagccgaga ttgcaccatt gcactccagc 11760
ctgggcaata agagcgaaac tccgtctcaa aaaaaaagag tggaagacaa tggtttccag 11820
ggaaaatggg atggtgtcat ctggaggcta gccatgcaaa aacaacagat gtcccatggt 11880
aatccagggc aaggctgagt cccagcattt tcaaatcttt aatgagaagt gcctgaaaag 11940
aaatgaagag aacttgtcac agtcccacct gaactggcct cttggtgcct cacacatagt 12000
gtcatttaag actaaataac tggatttggg ttattaatga ctggacttgg gaaacaaact 12060
gctgtcaccc tatgaagcga taggcttatt ggggctgcag agccagacct ccctctatgg 12120
gtgggagtag aattttctct gaaggaaata gatgatccaa ctgtggacca tccagggaag 12180
aggttgattg gagcagggaa tgaggattca ggaagctgac ggagctcaga caggaatgag 12240
gggaagctct tccgcctctc ttaaggacca tggttagctg ctatggagac gcctgaattt 12300
ttccattgct gtgtagggcg tgtcagccca gggctacatg aacacagatg agaagtgagg 12360
cagaaaagca ccaaacaagg ccaactttat gagtatgcca cctacaaagt cacataacgc 12420
cctgcactta gaagggccct gcatttggtt taacactttg cattcgccat ctttaaagtc 12480

ttaataatgt ttgtacaagg acccctgtat ttttatttta tatagccatt cttgctgcca 12540
gataaaaacc cctgcaaggt ctgagcctgg ctctgtgggg tagttttgag ctattccctg 12600
gggcacctct gccttctgat ggcctcacac cacgcctggt tttggatgtg cctcgagtaa 12660
ccagggaaga gtcatcttat gctttgggcc agatggcaaa ggagtgggca gcaccacagt 12720
ggcattcact gtcgaggggt ggttgcgtcc ccaggacagc atctgcgagg gtcacgggca 12780
gcaggaagtg tggcctggaa ggcagatcag acagtccgaa ggtgggtggg tgggcaagtc 12840
ctcaggggta agtggctgtg acagaggcag gggaagaagt agacagggat catgtttgtg 12900
atctgagcat tgtgctgaaa aaacaataag ttcatattgg tggctattag tgaataatga 12960
gagtcattat tttatttatt tatttattta ttgagacgag tctctctctg tcaccctgga 13020
ctgcagtggc atgcactctg cctcctctgc ctcctcactg caacctctgc ctcccaggtt 13080
caagtgatcc tcctacttca gcctcctgag cagctaggat tacagatgtg tgccatgaag 13140
ccctgttaat tttttttttt ttttttagta aagatggtat tggccaggct ggtcatgaac 13200
tccagacctc aagtgatcca cctgcctcag tctcccaaag tgctgggatt ataggcgtga 13260
gccaccacac ccagccaaga gagccattat tattaatata tttaattgca aaatttgaaa 13320
aatgaaaaac aaagaaaaaa tttcctgtga tgccagtgtt tcaaaataca tatcttaaaa 13380
ataaaaatgg actgcgcacg gtggcttacg cctataatcc cagcactttc ggaggccgag 13440
gtagctggat cacttgaggt caggagtttg agaacagcct gaccaacatg acgaaaccct 13500
gtctctacta aaaatacaaa aaagttagtc gcgcatggtg gcgggtccct gtaaccccaa 13560
ctactcagga ggctgaggca ggagaatcac ttgaacctgg gaggcagagg ttgcagtgag 13620
ccaagattgt gccactgcac tcgagcctgg gtgacagagc aaggttctgc cttagaaaaa 13680
aagaagatga aattgccatg taaaaataca tgcatgtgtt tatttataca tatgtgtgag 13740
atagatagat agatagatag atagatagat agatagatag atagatccat cataggaatt 13800
ttgttataca catttttacc ttcattcttt ttaatcacta catagaattc cattgtatag 13860
ataagtgaaa attcatggtt tattctgaaa ttttgaaact catagaaagg tacaaaaact 13920
agaagaacaa aaagaaaatg acacatgtac ctaccatcta tatttactaa atgctaacat 13980
tttgccttaa aaaaaaaaaa aaagaaggct cctataggta gctgggatcc actttgcaag 14040
atagccatgg gatagaaggt taattgggtt actttgtttt tgttttgata tggagtcttg 14100
ctctgtcacc caggctagag tgcagtggcg cgatctcggc tcactgcaac ctctgccttc 14160
tgggttcaag cgattctccc acctcagcct cccaagtagc tgggattaca agtgtgcacc 14220
atcacaccca gctaattttt tgtgtgtttt tagtagagac agggtttcac catgttgacc 14280
aggctggtct cgatctcatg acctcaagtg atctgcctgc cttggcctcc caaagttctg 14340
agattacagg cgtgaaccac tgctccaagc cctagtttgg ttactttgaa atcacagcct 14400
gatgggtctg cggcactttt agttacactc catttcgtag aagagaaaag agatctgaga 14460
gacattcagt ggttgttcaa gggcactcag cagggaataa aagagaaacc ccaatccaag 14520
cttcaactcc agaccctaac tcttctcctt atccctccct agattcccaa ggtccttagg 14580

aaagggaagg cttggactgg aagctccccc aaaccccttt atcactgacc tccagaaatg 14640
ctagtgaagc ccaggtgaca atctcagcct ggcccaggat aaacaccaaa gcaaaatcaa 14700
tgccagtggg agatattcat tcacaaagtg gattcaagac cctgcgtgca gagctgctca 14760
gatgaagcag gcccaaatgc agttagtcac cgttaattgg gtctcaggag tggaggagca 14820
aacaacagca tcccctgggg atttgcctcc atcaacccaa gtgcagggta agaagcccag 14880
gaaagcaagc caagctttgc aagcaccagc actgcccctg ggcagactct ccttcactct 14940
atctccacgt tctctcccat ttgccctcat atcttgttat attttttttt tcttgagata 15000
gagtctcact ctgtcaccca ggctggagtg cagtagtgcc atttcggctt accgtaacct 15060
ctgcttccag gttcaagcaa ttctcctacc tcagcatccc aggtagctgg ggttacaggc 15120
acacgccacc atgcctggct aattttttgt atttttagta cagacggggt ttcaccatgt 15180
tggccaggct ggtgtcaaac tcctgagctc aggcaaccca cacgcctcag cctccaaaag 15240
tgctgggatt acagacgtga gccaccaagc ccggcctatt attattattt ttgagatgga 15300
gtctcgctct gttgcccagg ctggagtaca gtggcaccat ctcagctcac tgcaacctct 15360
acctcctggg ttcgagtgat tctcctgcct cagcctcccg agtagctggg attacaggcg 15420
tacaccacta cacctagcta gtttttgtat tttttttagt agatacgagg ttttgccatg 15480
ttagccaggc tggtctctaa ctcctgacct caaatgatcc acccatctag ccctcccaaa 15540
gtgctgggat tacaggcatg agccatacaa ccagcctctt ctagtatttt aagctgactt 15600
actgtaccta catctccact ggttctttcc cttgccacct gccagacagc tggatgttac 15660
agtgcaagga tgcaaggtct ctgggtcgca gtccttcttt gcttattatt aattttgcag 15720
aggctgtgtg aagtttgaaa taagtgcacc gtctgaattt cacaactctt tccactgaac 15780
tgagggaagg caccgggcag tatagctgag atcagggcct gtgacctgtt gcttctttgt 15840
cgtggaaacc ctctgcgtca ttgatagagg gcagcattct gcatgtaaat gccaacaagt 15900
gccagtgctc tgctctgctc atagcagatt tctactacac aagggtgcag aatgtccctc 15960
ctgtatttct gaccttgggg gacagtcttt cccctccctt aattctgagc ccctcctcag 16020
aggtaccttc agaaaaacaa atgagtcagt caccttttct ttttatttat tcagtttatt 16080
ttatttattt attttatttt tatttattta tttatttatt tatttgagat ggagtcttgc 16140
tctatctccc aggctggagt gcaatggcac gatcttggct cactgcaacc tccatctccc 16200
aggttcaagc aattctcctt tctcggcctc ccaagtagct gggattacag gggcccgcca 16260
ccacacccag ctaatttttg tatttttaat agagatgggg tttcaccatg ttggccaagc 16320
tggtctcaaa ctcctgacct caggtgatcc acctgcctcg gcctcccaaa gtgctgggat 16380
tacaggcgtg agccaccaca cccagccaag tcaccctttc ttgatgcaag agttgcagac 16440
cctcaggaca gagccatgtg aggatatggt cacagtggac tactttgggg aaggggcttg 16500
tgctggggca gggggaatct gattgcttca agggtccctt tcacaactac tggagagctc 16560
cacagctctc attagtgaca accttcatca gcctccaaac aaagagggtc actagaggcc 16620

ctcaggggaa gcctcactac accagcggag atgtgatgga gcgggtccat gcctggagaa 16680
cagtgtgaac cggatccatg gctgccaatt ctcagtccat acctaagtac cagcccctga 16740
caagtaccaa ctgatgtgta ggaaacgata cacacacatc agtagatgtt ggcataaagt 16800
taccaactgt cctttcttag gaaatactgt ctattatcct gagacttggg gtgctcaaag 16860
ctcctttctt ttatgaaatt atggtgacaa tggattgttg aatagaaact ttgttaagca 16920
acccctcact taaccaactt attaggtcaa ccaataccct tcagtctctt actggagcac 16980
actgactaag cctgaggcac agagtgctca cagctggcag gctcttctct gctctcacag 17040
ttgtggtacc tggttaacaa gaatgcattt gggcccatac ttgcttatgc tggtcatacg 17100
tgttaactag aatgatgtac ttgaatcaat tcacctgtaa acaaaggaat tggccgggcg 17160
tggtggctca cgtctgtaat cccagcactt tgggaggcca aggcaggggg attacctgag 17220
gtcaggagtt cgagaccagc ctggccaaca tggtgaaacc ctgtctctac taaaaaatac 17280
aaaaaaatta gccaggcatg gtggcaggtg cctgtaatcc cagctacttg ggaggctgag 17340
gcaggagaat tgcttgaacc tgggaggctg aggttgcagt gagctgagat cacgccacgg 17400
cactccagcc tgggtgacag agaaagactg cacctcaaaa aaaaaaaaaa gaaaagaaaa 17460
gaaaaagaaa atacaaagga atttaaacag aaagactatt gaccaataaa ataagagtgt 17520
aaaaaaaaag agaacggtta tctctacaaa aactaaactg ctttggaaag acactataaa 17580
agcctgtcac tttttaaaat gctatcaaat taggaatgaa tgagagaact gtaaaatatc 17640
ggaaagaaat gcataaaaat gaagaaagat tttgcaatca ggtggcttca taagtttcta 17700
gaatttcttg cttcatgtta aagaaactgg aactgaaacc ccaggatcat gtgtgtgtgg 17760
tttgtgcttc aggaaaggca atgggcccca cttttcagca gacttgtaag caaagcaaag 17820
gctttaggtc tacatcaaaa tacttgctaa taactacaca tttagatatt ttaagtcaaa 17880
ataaaacatg tagaggaaaa atagccttgg cgcagtggct caaacccgta atctcagcac 17940
tttggaaggc tgaggcagat ggattacttg aagtcagttc aagaccagcc tggccaacat 18000
ggtgaaaccc tgtctctact aaaaatacaa aattaaccag gtgtgggcga gtgtggtagc 18060
tgacacctgt aatcccagca ctttgggagg tcgaggtggg cagatcacct gaggtcagaa 18120
gttcaagacc agcctgacca acatggtgaa gccccgtttc tactaaaaat acaaaaatta 18180
gccaggcatg gtggcacatg cctgtaatcc cagctacttg ggaggccgag gcaagagaat 18240
cgcttgaagt cgggaggcag aggttgtggt gagccaagat tgcgccactg cactccagcc 18300
cgggtgacag agcaagactc tgtctggaaa aaaaaagaaa aaagaaagaa agaaaagaaa 18360
aaatacagta tatctctttt atgagttccg ggtttaacaa actttttcaa tgaactgacc 18420
aattaccaat tcctgtgaaa ttgaataaga atttacctac ttggttcaga catatttgac 18480
tttggcaaag aaaactgaaa agctaaaaaa aatgtgccta cttggtagaa taaaaggctg 18540
atcactgtat ctgtccttgc caatctaaaa aatttattgg tacatgaaat caaccgattc 18600
cagcagtctc ccctttccaa ggctaagcag ctttgagctc tatttttcaa gatgtcaagc 18660
aaaggtagcc tttcacctca cttgagtgca aatttatctt actcatggat tttggaggtt 18720

gccaactcag tagttttttt ttctccctca actactctca aacagtacta tcttctgaaa 18780
gccaatgata tgcacatagt tctcaaaagg aggctgtcca aagcttacag gcttatcaag 18840
tggaatcttt tttgacatga ggcctcactc tgtccctgag gctggtgtgc agtggtacca 18900
tctcggctca ctgcagcctc aacttcctac gttcaagcga tcctaccccc tcagcctctg 18960
gagtagctgg gtctacagtc gtgcacaacc acacccagct gattttttat ttttggtgga 19020
gatatccttt ttgtaggcct ccctatgttg ctcaggctgg tctcaaactc ctgggctcaa 19080
tcgatcaccc accttggctt cccaaagtgc tgagattaga ggcatgaacc acgatgcctg 19140
gcctggaatc tttaaatatt aataattcaa aaaccaagaa gggaagcccc agcaacttta 19200
gatggctata aataattagg cactcaatca ggattgagtg cttagaaatc ttggagtttg 19260
ttttaacaca atgtgctttc ctggaaatca tataacaaga tagtaaatgc taccctcact 19320
cagacctgag gaaccatatt taagatggtt cctcttgcaa taaacgtgct ggccagccac 19380
ccaaattctg ccttagcaaa gtcatgatgg ggtgcgggga tacggctgag atgacaccca 19440
gatacaaatc tatagacatt atgaaacaat ttcctttcct gcctcttgcg tatatctgcc 19500
tccctcctct cgccccagct acttctctag cactctaaac tctagctcag gcttgagcaa 19560
accgggctgg attagcagct gactcctaca gtcatagcac tgcacagaga gaacccagac 19620
acttcctatc aggaccacaa ggcagagatc tgctctgtcc agctctggaa ttgactggac 19680
tggctcatga ctgtcaacct atttctgaga acataaaaag gtccctttca gactgccacc 19740
aattcagagg gctagaattt tagataaaaa cagtatatcc agtaatctgt tctctcttgt 19800
agacacttcc tcgttttttt gagacagggt ctcgttctgt caccgaagct ggagtgcagt 19860
ggcatgatca cagctcactg ctgcctagaa ctccagggct caactgatcc tcctgcctca 19920
gcctcccaag tagctgggac cacagacgcc tggctaattt ttttaatttt tttgtagaga 19980
tagcaatcct caagcaatcc tcctacctca gcctctggag tagctgggac tgcaggcgtg 20040
cgccaccaca cccagctaat taattaatta ttgtaggtct ccctgtgttg cccaggctgc 20100
tctcgaactc ctgggctcaa tcaatcacct gccttggcct cccaaagtgc tgggattaga 20160
gttgtaagcc accatgccta gcccagaatc gttaagaatt aataattcaa aaaccaaggg 20220
agggaaaccc cagtaacctt agatgggttt atcacgttgc ccaggcttgt ctcaaacacc 20280
tggactcaag tgatcctcct ggctaagcct tccaaagcac tgggattaca aacgtgagcc 20340
accatgcccg gcctgtagac accttttttc aaagtttgac taaggcaacc aaaagggatc 20400
agaatggatc ggtcttccca tgctgtttca ctgctcttca gcagcccatg tccaccatcc 20460
agatgaaggt agaagcagcc caattcctcc agccaagcca aattgagcta aattctagtc 20520
ttccctgtgc gtctagctta atgtcagaag tttcatggca ataatcttca ctgggatcta 20580
tgattttata ttaccagaaa agatgaatcc gtttctatcc aacataagca tatacttata 20640
cattctgggt agtgctggct gcttgaaata aactctgaaa taaatccact ttatgcacaa 20700
tagttgaaat ggcacagtta cgctctgatc taaggcctta tgttatgcca tttttttttt 20760

tttttttgag acggagtctt gctctgtcac ccaggctgga gtgcagtggc gcgatctcag 20820
ctcactgcaa gctccgcctt ctgggttcat gccattctcc tacctcggcc tcccaagtag 20880
ctaggactac aggcacccgc caccacaccc agttaatttt tttgtatttt tagtagagac 20940
ggggtttcac catgttagcc aggatggtct cgatctcctg accttgtgat ccgcccacct 21000
cggcctccca aagtgctggg attacaggca tgagccaccg cacccggcca tgttatgact 21060
tttattctta taccttgact cattctaatt tggctctgct ttgaactttt ccattttaag 21120
atctctctgt gggcgcttct tgctctgtaa atttaaaaca gtctgtgtca aaaacagtct 21180
cactctgacc tgaccagctt agatgactag ctcaagaata gtcaagcttg cttgggctcc 21240
atgaagtccc ccagagcgga aaagcccttg aaattctagg gaatgatctt tgaggtcatg 21300
ggagcaggat ctctccacca aggtcaccaa tgccagatgc ccttggggtg tccatagctg 21360
tgagaagtca ggcaaacccc aggacacatt gatatggact gctaaacatg cctacagcct 21420
cctacctgct gggtcaccgg cctgtggtgc tcagggatag ctcgtcatct cttcccaagc 21480
catgatgcct ttccctgaca ctttatcccc agcccctccc tacaatcccc ccatccaaca 21540
aaaatcaacc tagatgaagt gatagacctt tgtcctccag ccagaggaca tcgtagcccc 21600
tcatctctcc tcaattttca ggagaggaaa aaggagtctg tccacgtcat gcttctgggc 21660
agtcaatcct gaaattcctt aggttgtttg gcccaggact gccctgccag aatagtgaac 21720
accgctgcct gtgctctgag agatgccttc tactccaaat acactgctta ttcattgagt 21780
caaccaataa acatttgaga acccatcatg tgccagacag taactaggca cgggggatat 21840
gggagtgaat gagacaaagt ccccacttaa ggcaccacac agcctagtgg agatggggaa 21900
aatgacaaac acagacccca ctacataagt catttcacca aaggaaataa agcggagcaa 21960
caagacagag agaaattgga ggtgggctgc aggggccact gccccctaag tggccaagga 22020
aggcctcttc taagaggtaa catttaagct gaaatgtgat gataaaaagg agctaactat 22080
tcaccagtct gcgggaagag cattctggaa aggaggaata gcaaatgcca aggccctaag 22140
aagggagcaa gccttatctt gttagaagga cagaagaaaa gctggtgtgc atggagcaga 22200
gcctttgaga gggagggtgg atggagttgg gggtgaaggg gcagccagca gcccatggat 22260
cccctgagaa cttgcaggct gggagagaaa ccatcagaag gctataagca gggagtaatc 22320
aggtctgtgc ttggaaaaga tcactctggt tgctgggacg agaatggact ccatggcaga 22380
gaatgaaaga ggaagaccag gcagggaatg gttgcttgga ctagcaatca ttgtgggctt 22440
ggtaaccaca gagatgatga gacgtggtgg ggttcagggt actttctgga aggaaggctg 22500
tcagattgct aaaataaaga taataactct gtctgggtgt ggtggctcac acctataatc 22560
ccagcacttt gggaggtcga gataggcaga tcacttgagg ccaggagttc gagaccagcc 22620
tggccaacaa ggtaaaatcc catctctact aaaaacatga aaattagcca ggcatggtgg 22680
caggcacctg tagtttcagc tacttgggag actgaggcaa gagaattgct tgaacccggg 22740
aatcagaggt tgcagtgagc cgagatctca ccactgcact ccagcctggg gaacagagtg 22800
agactctttc tcaaaaaaaa aaaagatagt ccagcatctg taaatgagca tgatccctgt 22860

gtgagcagca gtgtatgtgt atacacacac acacacatgc agacacatgc acatacacac 22920
tatcctcctt atacaaacag tgcagactcc aggctctgga gtcaaatgtg ttcaaattcc 22980
aactcatctg cttacttgcc attaaacttg gttcaagtca ctgaacctct ccatccctag 23040
ggttctctgt ctgtaaaaag gggacagtaa tagaagtgcc tcatggttgt catgaggatt 23100
aaattagtta ctacatggga agcatctaag gaagggcaac tgccacatac cgtatgagtt 23160
tcctgttgct gctgtaacaa attagcacca atttcgtggc tcattaaaaa caacacaaat 23220
ttattctcct gcaattctgg aggccaggag ctcaagatca gtttcaatgg gctcaagtca 23280
aggcgtgggc agggctggtt tcttttggag gctccaggca ggggaatcca ttcctttccc 23340
tttctggcat ctgaaggctg cggcattcat ggcccctgct cacgtcacac ccacgtccac 23400
ttccatcatc actttgtctc tacctctttg accttctggc ctctttctta tgaggaccct 23460
tgtggtgata ttttgggccc acctgaatca gcaagaataa cctctctatc tcaaagtcct 23520
taacttacat acatctgcac tgtccctttc tgccacagaa ggtaacagtc acaggttctg 23580
aggattagga cacagatatc tttgggggcc attattcagc ccataacatg tggtaggcaa 23640
taaataaagg ttcattgtat tacgataatg gtacttcatg ataataatga taacattttt 23700
ctcattatca tcttcatcat tcccatgaaa gccccagagc atatccctag ccccctcctc 23760
aactatgtag gagaggaggg gacgtttaga aaaccccaac tcctagaagc cccctgagac 23820
ttggccccaa gcccaaccca gagctagggg caatgctggc tgatcaggca gaggcaccag 23880
tccccgggtt ggtcctgatg aggggagcag ggggcagacg gtcgatgagg caggagtgtg 23940
ctcacagcct ggggtccaag aaggagcctg gagtgcaggc taaggcctgc tgatggtgct 24000
gtattccgtg ggctcctcag ggcccctgcc ggggaggtgg ctactgaggt ggcccatgtt 24060
gcagtaggtc ggttcctgat cctcagcacc caaggtcaga gatgcatagg aaatgtcctc 24120
cttcggcaag gaagcctgca gcaaaggcgg gctccaggtc agagaggacg gcaggaagtg 24180
acggtcacgg ggcagaggag ccctcggagg aggatagtgg ggatggacca ttcagggctt 24240
ctaatcccac tcaagccctc cagcttcccc cttcactctg ccaagcaccc tgagcttcaa 24300
ccacaacaga acagattctc ccccaaccct gcaccagtgc agcacatttc ctgtggaatc 24360
ccaagtcact cagcctgtct gggactctgc tgcccacgtg gggactgtgt ttgccagtcc 24420
ctgtgtcccc aggactgaga cttgcctgtg ggagctccta ggcgagtcct gcagtgacta 24480
tggctttgcc ccacacctgc ctcctctatg cccacatgag caggagaaag cccaccctcc 24540
aacggggtgc aacggcaggc ctgtgtcccc ctgccccagc ctcacagcag cccccatgga 24600
ggacgcacca tggtgacata ttccacttcc acctggtcaa cctgggcaga ggaaagcttc 24660
gtggtagcct tttgcgggga ggttccggcc agctgcaggg tcaggtctgc atagcagagg 24720
tcgccctcca ggggctgcag tacctgggac agggaacaca ggctggggag tcacaagatc 24780
tgtctgtgga cacaggttcc ttttccacgg tgggacggga cgagagcctc tccacttccc 24840
caggtgcccc tctcccattt ccctccgtgt cctccacttc tcaggctcct gtacccctca 24900

gcccttgtcc ccctggctgt ttccaggccc tcagccccca ggccctgcag ggtgccatga 24960
ggacaggata ctttggcaca ggacttctct gatattttgg accttctgag agatggaaaa 25020
tgaggaaggg gaaagaagct gcctggtctc tctggtccga ccttgggggc tatcttacct 25080
gctctgggga catcccggct gctaaaagac aaacaacaat tcagaattag aaagcccatt 25140
ccccagactc acaagaaccc ccaggtctag ggaagagaaa tatggaagaa aagaggtgaa 25200
ttgggtccag gctcagagac agggacctgg ttctaattct gattcctcag atgacacagt 25260
ctgtgacatt cgtcatgtca cctctctgac ctttcacttc tccaccagga aaagagggac 25320
accccagccc cctgctcccc acatcataga actgtgcact cagctgatcg ggtggccctg 25380
ccagccatcg gctcccctgg gagtaagttt gcttctgcag aatcccaact cggcctggaa 25440
ttccaccatg tctctctacc tgattcatcc ttcaggggtc aattcaaagt catgcctgcc 25500
ttctgcagct acccaggctg tctaatcccc ctcccgggcc cccggggggc acttgctcta 25560
tgtctgtctc atcatcttcg tcctactgaa ttctagttag tcctcaggtg ggctgtaagc 25620
tgcagggtgg ctgggcctgt cccttcctta caacctgggt gcttggctgt gctgagcagg 25680
tggtgctcca tcagtaaaag ttgtctgcag agacaggaat gcaccaagct gtcccccaac 25740
cccgagtaga atgtgcaagc tgggtcggca cagtagggac cgattttgtt tggtttggtt 25800
tggtttggtt tggtttggtt tggtttggtt tggtttggtt ttgagatgga gtttctctct 25860
tgtcacccat gccggagggc aatggcacca tcttggctca ctgcaacctc cacctcctgg 25920
gttcaagcga ttctcctgcc ttagcctcct gagtagctgg aactacagat gtgtgccacc 25980
atatccagct aatttttttg catttttagt agagaagggg tttcaccatg ttggccaggc 26040
tggtctcgaa ctcctgacct caagtgatcc acccgcctca acctccctaa gtgcggagat 26100
tataggcatg agccacgaca cccagagatt ggattggatt gtttttccac tgggaccgat 26160
tttcaggtgg ttgctggtcc ctgtcttctt tctcccctcc ccagccacca gccccacatc 26220
cctgccctag ggacaacaaa gaaatggggg gttttcccct ggtttcaaaa gtctagatgc 26280
tggagaacac agaaagcact tagaccttag gccgtgggca atacatttgg gagaaaaagc 26340
gatgaataaa agtcaggctc taattttgaa aaagcatcac agaaaacaaa ctaacagaga 26400
gtcaggctcc cctcttccca ggcgaaagat ttcccaggct gtggggaggg agaagtgttg 26460
gagggaaccc tgtgagagtt aggacccagc tgttctagaa gagaaagagg tacaattgtg 26520
tgagaatgtg gaagtgaagc cacattctca cacaactggg gttttgttga ggccaggcct 26580
catgatctgt gagtgtagac tcctgcgcca agaggctcct ccctgaagag ctagttcaca 26640
aagggcaagt aggtcagcct gggaaagtcc agtgggcaag ggagggacag aaggaaaggc 26700
cattccaatt tcctgatcag acttggaggg ctgtgtgtgt gtgtgcatgc ttgtgtaatg 26760
tgatgagtgc acatgcactg tgggatccat gcagtgtgtg tgcccatgat cgcgcatgtg 26820
tgtgtgcatc cactggtgtg tttgtgtctg tttctccctc cccattccat gtcagctcag 26880
tacctccttg acaaccacat agacccaact ctagcgtcta gtctagacac cccagactgg 26940
accacccacc taagaccatt gccacctatg ccatcaagag tcaatccagg aaagagacag 27000

ggaggtggat cagagagaag agaaggggaa ggacggaggt gcagggcagg cggcgaatgc 27060
caaaacctcc tggggctccc ctgcaagctc cagggcgccc cccggtcccc tggcactgcc 27120
agctgctgag ggcctttggg actgtgccat ttcctgattg tgtgggtaat cccttggacc 27180
agatagctcc cagatgccac atccccagcc acccggccca gtctcagccc agcctcaccc 27240
aggtcctctc acctttctgc tggtacttca tcatcctcca agccaagagt gaggcggcca 27300
ccaaaagcag cagcaatatg gtgaagatga ggggcaggag gacactgagc ttcaggagct 27360
tgtgcctaga aacaatggca agcgtcccct gcatcccagg ctcaccacct gcctctcagc 27420
ccaatcccac ccacccagca gcaggggagg gccacacacc tgatgagctg caggtctgta 27480
gtttgcagcc tgggaaccct cactcctggg gatcctcaaa gatgggttta caatgagtac 27540
acatagacac aaagaaggga atggaacaag agacaccagg gcctacttga ggatggaggg 27600
tgggaggaag caaagaatca aaaaactaca aactacctat tgggtactat acctattacc 27660
tgggtgacaa aataatctgt acacaaaacc ccggtgacgt gcaatttacc gatataacaa 27720
atctgtacgt gtacccctga agctaaaata aaagttttta aaatttaaga attttaacaa 27780
aagatagttg tttttggttt tctttgtttt tcattttttt gacacagagt cttgctctgt 27840
cacccaggct ggagtacagt ggcatgatct cggctcactg caacctccac ctcccaggtt 27900
caagagattc ttctgcctca gcctcccaag tagcggggat tacaggcacc caccaccaca 27960
cctagctaat ttttgtattt ttagtagaga caggattttg ccatgtaagc caggctggtc 28020
tcgaactcct aacctcatga tccgcccacc tcagcctccc aaagtgctgg gattacaggc 28080
gtgagccaca gcgcccggcc ttaacaaaag atagttttta aaaggagaaa agggttccat 28140
gaggtggttt taatgtgtct agaagcctaa cagaaattgt agggcagcag actcctaaaa 28200
cacctcctgc ctctacacct gccctctcta ctgggttgaa tagcgtcctc ttcccctcga 28260
attcacatcc actcagaccc gtgaatgtga cctatttgtg gatgtaatca agttaagatg 28320
aggtcatagt tgattagcat gggccctgat ccagcgactg gtgtccttac aagagggaaa 28380
tttggacaca taaacacaca caggaagaag accatgagac gacagagaca gggatcagag 28440
tgatgccgcc acaagaccgg gaacacaaag gatcgcaggc gcccaccagc agctggaaca 28500
gaggcatgag acagactctc cctttgagcc cccagggtgg caacagccct ggtgacacct 28560
tgatttccaa ttcctggcct ccttagctgt gagagaacgt atttcttttg ctttgagcca 28620
ctcgtaacgg cggccacagg acactgacag gccctactgt agtggatcat tgccacagca 28680
gccgggggcc ctgtgaacca aacgtcaaag cacacccctc tctttctcaa aattctgcaa 28740
caggccaggc acagtggctc atacctgtaa tcccagcaac ttgggaggct gaagtaggca 28800
ggtcacttga ggtcaggagt tcaagaccag cctgaccaac atggtgaaac tccgtctcta 28860
ccaaaaaata caaaaattag ctgggtgggg tggcgtgtgt ctgtaatccc agctacttgg 28920
gaagctgagg tgggagaatt gcttgaaccc aggaggcgga ggttgcagtg agctgagatc 28980
gcgccactgc actccagcct ggtcgacaga gcaagaccct gtctcaataa ataaataaat 29040

aaataaataa ataaacaaac aaacaaacaa tcaaaataaa ataaataaat aaataaatcc 29100
cgtaacagcc ccatttcagc ccatgaaaat ggccagggct gacaatggcc tccactcctg 29160
cctacgactc acccctgcct gcttcactcc agcctcactt cctgctgctc ctcaaatatt 29220
ccaggcacat ccccacatta cggaccttat tccagttgca tttttctcca ggaacatgct 29280
ccctccagag ctctgtgtgg ctcactttct cacctcttcc agtctttgct caagaatcac 29340
tcgactgagc gcggtggctt acacctgtaa tcccagcact ttgggaggcc gaggcaggct 29400
gatcacatga ggtcgggagt tcgagaccag cctggccaac atggcaaaac cccatctcta 29460
ctaaaaatac aaaataacag ccgggtgtgg tggtgcattc ctgtaatcct ggctactccc 29520
agctgcttgg gaggctgagg caggagaatc gcttgaacct gggaggcgta ggttgcagtg 29580
agccgagatc ctgccactgc actccagcat gggtgacaga gcaacactcc gtctcaaaac 29640
aaacaaacaa caaaaaaaac tgttgtgggc tgggctggat tgtacaccac caccccccca 29700
ccccaatcca tatgttgaag tcccaccctt tagtatctca gaatgtgatt gtatttgaag 29760
ataggatctt taaagaggtg attgaggtta agtgaggtca ctggggcagg ccctaatcca 29820
ttatgactgg tgtcattaga agagatcagg acacagacac acgcagaggg acgaccacgt 29880
gaacacatag gaaaaagata gccatctaca agctgagaag actcagaaga aaccaacctt 29940
gtggaccact tgatctcaga cttttagcct ccagaattgt gagaaaataa atttttgtga 30000
tttaagcccc cattctgagg tattttgtta ggggcaaccc aagcaaacta atataaggat 30060
tatctccttc atcactggat gtgcagaaat ttgagaaaga aattcatcag tcaattaatt 30120
gtgtcaaccc tatttggtta cactaattag ctctagattg taagtacctc gagggcaggc 30180
attttgatta tttggtccat tcttgtaccc tttttaattc ccttaaacag tgacaggcta 30240
gagtgagtgc tcaagagtta tgtgttaaat aaatgagtga acaaaccatc tcatgctaat 30300
caaaggccct aatcgggcac ttacataagg cttcgattca taaaataata aataaataaa 30360
taaccttaac tattacttat tcaaagcagt ttggtagaaa ataaactttc aaatcagagg 30420
gtatcctgta ttgatttgct taggaaaaag aaaaataggg acaaagcaag gtggcttatg 30480
cttttaatct cagcactttg ggaggctgag gtgggcaaat cacttgagga caggagttgg 30540
agaccagctt ggccaacata acaaaacccc atcgctacta aaaattggcc gggcatggtg 30600
gcttacgcct gtagttgcag ctactcgaga agctgaggta tgagaatcac atgaacctgg 30660
gaggtggaag ttgcagtgag ctgagattat actactgcac tccaacctgg gcaacagagt 30720
gagaatccgt caaaaaaaaa aaaaaaaaaa caattagctg ggtgtggtgg tgggcgacta 30780
tagttccccc agctactcag gagactgagg catgagaatt gcttgaacat gagaggcaga 30840
ggttgcagtg agccaagatg gcaccatgca ctcaagtctg agctacagag caagactgtc 30900
taaaaataaa aaaaaagaaa actttaaaaa gtttaattta aaaaaaaaac agaaggcaaa 30960
tgggagaggg ggagtagaag ggtctcaggt ggttagaata ccggggacct cccataggga 31020
ttttgtggca aggtcccagc aaaaggagaa cttggcactt ggcagatcta aaaacaaagt 31080
caagagggcc aaggagagtg aaaggaagaa attaaagggg acgttcctct tctagaaagc 31140

ccagctgact gcagatggtt gggcagggca ctaagaggca tgacctgagc tatggtgtag 31200
agcccagaga cttccatcca agctccagca ccacccttca ctgaggaagg gactgaggct 31260
cagtttcttc ttctgtaaaa tgggaacatt tgtatctgcc cattcttcca catggggtgg 31320
ttgtaggaaa ggtcaaagaa aacaaaataa tgcacaggaa agccttttgt gagctgtcag 31380
ttgctctaga gatgtgagaa actataggca tcactgttct tgccataata gtaatcagag 31440
gaaaattaag ggaaacagga cttccccaag ccctcctttc cttgtcctcc cagaagttga 31500
accaagagtc atccctgcgg ctccatgacc ccacgcccac actcacatga gacacactgt 31560
cctcactgac tgccaactca tcggtctccc acactgagct ccacggggac agtccatgtc 31620
tgtgtcccca gagctcagca cggtgcagcc atgtagaagt tattataaca cagtaaaagt 31680
ggggcgaacg aatgaatgaa cctacaaaca gcactgagct gcagaatgaa ggatctgtgg 31740
agactgaaag gacaccatgt ggcctgacag caagtgggaa gggctcccag cttcctcatc 31800
ctcaccaagg agcatgcagg tcccagacaa agctcaggct ggaaaatggg acttcaaaga 31860
gttctccatt ggaggagttt gggccaagta ggccacagtg gaaccagagc tggcttacct 31920
gttgtccaag tggtggccgg tcagagttgg ggagctgcta gtttcttctt gggtgactgg 31980
tgctgtaaac gtagtggagg taggcgtggt gggggcagga gtcgacgctg tagttgatgc 32040
ttggtgtata aaccagatca cagatgcccc tgcccatgag cccacccgca gccctgcaca 32100
cagctctgag cctgggcggg ggtctggact gctactatgg gaaggggctg cagcctggac 32160
cactcatgtc tccctggtct ctaggagact ccagagcctc ataggcaggt ggggctgggc 32220
agggtccagg gaggacgctg agctggccct gggagggtta gagggggctg ggaagagcct 32280
aagggcagct ggagaccagc tgaacttgag caacctgctg cttatgacag tgccttggtc 32340
aatctccacc cccagcccta tttatccagc aaaaacagga gcagagtggg aacttgcaca 32400
gtgaccctag atctgtgaca tcaaaggtca ggaccagccc tgaatgaaat catcatattc 32460
aggaagaact ttgactttgg gcaagttaca aaacttcaat gatggggatg cccccttctc 32520
aggggtattg tgaggaccca ttgagacaat gcacgttcag cactttgcaa actagaatat 32580
gccccagagg ctccgggtgc aacctggggc cccaaggact gtggtgctcc ttccatgcca 32640
caagggccct cgagccatcc tgcttatctt ctgagggaca gcagaacaag cccaacaaat 32700
aaacccatgc aaaccgaggc actgggaccc ctgagctaac gctcccagga gtgtctgcat 32760
tctagaagga cataggaaga aatatcatta agtccacaca cacaaatggc tcatggtggg 32820
atgtcaggga ccaggaatgc agccgtttga ctgtgaagac ctttctggac ttgggaatta 32880
gaccccagca ccccgactat gctgagcctc tttttccccc aggttagata tggaatggga 32940
gatgggaaca gcagctatga agccccagcc cagaactcca ccaaaagcag gacacacctg 33000
ggcaccagag gtctccaaaa agcaatggca aggactgggg tctctgggta ccatgggaag 33060
gcatcccttt ggcccatggg ctgaaactgg gaccatcacc ttgtcctcag gcactgagct 33120
gtgcagaggt aaaaaatgtg cctagaaagg cttaatcccg tcgtggaggg agccctggga 33180

gccatggggg cattgaagag aggccctgct cccccagtcc caggtggtca gtcagggttc 33240
tatgagactc gggactcaag tgatagatca ctcagtgaca attaaatcac ttgagtagga 33300
cctcagagac caggacagag caggccctga gagacacaca catatataca ctccctctta 33360
cctgggtcaa tggtcacttg aactgtgacc ccaaggtcat ttccagtttt ctcaattcca 33420
caccagtaag tgtcagcatc agttttcatg agatcctcca tggtcacagt gaacgtgcgg 33480
tttttctgat tgtccttgat ggacacccgg tccctcttca cctcctgctc tgacccactg 33540
gttttaacaa ggatcttgca gtcacgccaa atagctcctc gacaccacca cttcaagtag 33600
gtctcccagc ctgatctgta aacacactgc acggtcaagg agccccgctc caagccattc 33660
actgttgttg gaccggtgat ttgagtgaca atggagtagc ctggaaaaca caaattcatg 33720
tgctgtcacc tcccacccca agggcagggc cacagctttc tgtttaggga atagctccca 33780
aatccaagtt tcacaggtta aataacagtc aaggaagaaa tacaccttcc gcagacaaaa 33840
cttttgtcct tcagctttcc actgggtcaa agccaccagg aaaatcactg aagagcacaa 33900
caacggatcc ccccaaaaat aaaaatattg gcataagcaa taataacttc ctgcagttca 33960
cccctctctc caccctacgg ccaaggtaat ctgtatattt caaggaatgt ccttttgcag 34020
ccatctttgt gttagtccag tgtggtgggg aggaaatacc aggagacccc tttccacagg 34080
gtcttacctt gactcttagc actgatgacc ctcatgaggt cgagctgagg cagccacatc 34140
agatggctgt ggagaggctg tcctggtggt cctaatgatt tctagtggtt ctggaataaa 34200
ccaccctgga tgaatgcctt tgaagttgat caaaacagca acggcaactg gtctccatgt 34260
gctataggat ccatacaaca cagatcccac tttctttaat gaattgcttt gacccctttg 34320
tcaaattgca aatttgtgga tttcccattc tgttccattg atctatttgt ccacctttct 34380
gccaatccta cattctcctg attactatca ccttgtagaa aagcaggtat tatagacagt 34440
aaggcagtat ttactttttt ttttcttttt ctcttttttt cttttgaaac aggctctcac 34500
tctgtcgccc aggctggaac acagtggcac aatcatggct caatcatggc tcactgaagc 34560
cttcacctcc caggcttaat tgatcctccc acttcagcct cccaagtagc tgggactaca 34620
ggcacatgcc tccacgcctg gctaattttt gtagttttag tagaaacagg attttgccat 34680
gttgtccaag ctggtctcaa actcccgggc tcaagcgatc cacccacctc agcctcacaa 34740
agtgttggga ttacaggcgg gccccacaac gcctggcctg tattttctta aaatacatgt 34800
tttccttcaa aatcatgtcc tttgcagcaa catggatgtg gctggaggcc attatcctaa 34860
gtgaattaac gcagaaacag aaaaccaaat accgcatgtt ctcacttata agtgggagct 34920
aaacattggt tatgcatgga cataaagatg ggaacaacag gcagaggagg gaaaggggca 34980
agaacaaaac agaggaggga agggagcaag cactgaaaaa actaccaatt gggtaccatg 35040
ttcactatct gggtgacagg tttgattgag gctcaaacct cagcatcatg caatatatcc 35100
atgtaacaaa cctgcacagg tacccactga ctctaaaaat aaaaaacatt ttaaaggaaa 35160
aaaaaaatat atatatatat atatcccaaa gtgctgcgat tataggtgtg agcaactgtg 35220
cctggccttt tttttttttt taagtaaaga aatatttccc tgtatccgga atctttcaaa 35280

ggtgagaata tgagagaaag aataaaaaga acatatatat ataaattccc aacatgaggt 35340
tgggagttcg agatcagcct gaccaacatg gagaaacgcc atctctacta aacatagaaa 35400
aattagcctg gcatgatggc acatgcctgt aatcccagct actcgggagg ctgaggcaag 35460
agaatcgctt gaacccggga ggcagaggtt gcaatgagcc aagatggcgc catagcactc 35520
cagcctgggc aacaagagca aaactccatc tcaaaaacaa aacaaaacaa aacaaaaacc 35580
agcagttcaa gcacaacaac caacggcgcg gacagagggc ctcagtgtca gagagaccct 35640
gggaaggggt gaggaccacg gcagcctgga ttggagagca gggtatccat gcgaaggcac 35700
agccctcctt ccactccagc ccgggtcctg gggaaagaag cgggccctgt gacaagaaca 35760
cacaatgggg aaaggacagt ctcttcaata aacagtgctg ggaaaactcg acatccacgt 35820
ccagaagaat gaagccggac ctttatctca caacatatac aaaaatcaac tcacaataga 35880
gaagatgtaa atgtaagacc tgaaacttaa atctctcaga agaaaaccta atggaaaagc 35940
tccacaacat tggtcttggc gatgatgtct tggagaggac gccaaaagca caggcaacaa 36000
aagcaaaact agacaaatgg gactcacaaa accaaagagc ttctgcacag caaaggaaac 36060
aaccaagaga atgaagcgac aacctacaga gtgagagaaa atatttgcaa accataaatc 36120
tgacaaagag ctgatatcca aaatacataa ggaactcaaa caaatcagta acaagaaaac 36180
aaataatcta attaaaaact gggcaaggaa tctgaccaga tatttctcaa aagaggagct 36240
ataaatgggc aacagatcta tttttaaatg ctcaacatct ctgatcacca gaaaaatgca 36300
aattaaaacc aaaagagggt caggcacagt attcacactt gtaattccag cactttggga 36360
ggccacattg ggtggatcat ctgaagtcag gagtttgaga ccagcctagg caacatggtg 36420
aaatcttgtc tctactaaaa atacaaaaat taaccaggtg tggtggagtg cacctgtaat 36480
cccagctact tgggaggctg aggtgggaga atcgtctgaa cctgggaggc ggaggttgca 36540
gtgagccaag atcgtgccac tgcattccag cccgagcaac agagtgagac tccatctcag 36600
aaaaaaaaaa aaaaattaaa agtggtatat gactgtatat cctgcacata ttaaaaagtt 36660
aataaaccat gaataagata aaaggatata ataataaaag gatatttaaa caatgttatg 36720
ccaatgaatt tacaatttga gatggaataa acaaattcct agaaaaaaat tatacaggcc 36780
aggcactgtg agaggccgag gcaggtggat cacctgaagt caggagttca agaccagcct 36840
ggccaacatg gtgaaacccc atctctacta aaaatacaaa aattagcctg gcgtggtggc 36900
acacacctgt agccccagcc acttgggaga ctgagacagg agaatcgctt gaacccagga 36960
ggtggaagtt tcagtgagcc gagatcacat cactgcactc caggctgaac aacagaacaa 37020
gactccatct caaaaaaaaa aaaaagaaaa aagaaaaaga aagaaaagaa aaaaaataca 37080
atatgctaaa atcagcacaa aataaataga aaacctgaat cattccacag ctattatatt 37140
taaataaatc aaatcccttg tttaaaactt ccgccagaat aaaactccag acccatacag 37200
cttcaatgga aatatttacc aaacacgtaa agaagaaata atgtctattt tatagataat 37260
tttccagagc atagaaaaag aagatgaaat cccaaatcca tttcacgaga ttaacataat 37320

cttgatgcca aaacctgaca agggcaaagc aagaaagtat tgtaggcaaa tatcactcat 37380
taacataaat gcaaaaacct gaaacaaaag atcagcatac ggaatctggt gatacataaa 37440
caggataaaa cattatcacc aagttgggtt tatgccaaac acccaaaatg gtttttaaca 37500
ttaaaaaatc aataaggagg ctggacgcgg tggcttacac cggtaatccc agcattttgg 37560
gaggccaagg cgggcggatc aggaggtgag gagatcgaga ccatcctggc taacacggtg 37620
aaaccctgtc tctactaaaa atacaaaaaa ttagccaggc gtggtggcgg gcgcctgtag 37680
tcccagctac tcggaggctg aggcaggaga atgacgtgaa cccggggggg cggagcttgc 37740
agtgagccga gatcgcgcca ctgcactcca gcctgggcga cagagcaaga ctccgtctca 37800
aaaaaaaaaa ggaaaatcaa taaggaggcc aggcacggtg gtttacacct gcaatcccag 37860
cactttggga gtccgagtca ggcagatcac gaggccaaga gatcgagaca atcctggcca 37920
acatggtgaa accccgtctc tactaaaaat tcaaaaaatt agctgggcgt ggtggcgggc 37980
gcctgtagtc ccagctgctc gggaggctga ggcaggagaa tcgcttaaac ccaggaggcg 38040
gaggttgcag tgagccaaga tcacaccact gcactccagc ctggagacag agcaagactg 38100
actcaaaaaa aaaaaggaaa aaagaaaatc aataagggta atatgccata ttaacagagt 38160
caaggagaaa aatcttgtga tcatcttagg tgcagagaga agtagtgata ggaatcaacg 38220
ctcattcatg attttttaaa aactcttagc aaactaagag aactgccttt atctgataaa 38280
gattgtctat caaaaacctc agcaagcacc acacttagtg cgagacattc atatgtactt 38340
tataataatt tgttaacatg gatgttgaag ttttatgcac ttttttgtac attttatttt 38400
ccacaattaa aaagactttt aggacaccat aaaccaagag ccaaattatt cttttttttt 38460
tgagatggag tcttgcttgg tcgcctgtca cccaggccgg agtgcactgg tacgaacatg 38520
ggtcactgca gcctcaagtc ctgggctcaa gtgatccttt tgctgtaacc tcttatgtag 38580
ctgggacgac aggaccacag gcatgcacca ccataccagg ctagtatttt tttttttgga 38640
gagacaaggt ctcactttgt tgcccaagct ggtctcaaac tcctggactc aaccgatctg 38700
cctgccttag cctcacaaag tgctgggatt acaggcttga gccatcatgc atcaccagcc 38760
aaattattct taacagtgaa atactatagg cattcctatt gacatcataa acaaaacatg 38820
aattcccaat atatataata acatgattct ggaagttctt ttttgtttgt ttgttttttt 38880
gagatggagt caggctctgt cgcccaggct ggagtgcagt ggctcaatct cggctcactg 38940
caagttccgc ctcccgggtt cacgccattc tcctgcctca gcctcctgag tagctgggac 39000
tacaggcgcc caccaccacg cccagctaat tttttgtact tttggtagag atggggtttc 39060
accgtagcca ggatggtctc gatctcctga ccttgtgatc cgcctgcctt ggcctcccaa 39120
agtgctggga ttacaggcgt gagccactgt gcccagcctg gaaattctaa tcaatatagg 39180
aagataagga aaaacataaa atgtataatt atcaaaaggg aaaaaagtta gagaaaattt 39240
tttaatttta aatgaaaaag tattttaatt cataattgaa tttaatagag tggctaaata 39300
ataaacatgt aaaaatcaat ggctttccta taaactgcta ataaatagtt ggaaagtaaa 39360
gtagaaaagt gctcatttac attagcaata aaagtgtatt aaattcctag gaataacttt 39420

aacagaaaca ataaagactt atggaaaaaa agcacagtga ggctgggcgc ggtggctcac 39480
gcctgtaatc ccaacacttt gggaggccaa ggcgggtgga tcacttgagg tcaggagttc 39540
gagaccagcc taaccaacat ggtaaaaccc catctctact aaaaatacga aaaaaaaaaa 39600
aaaatagcca ggcatgctgg cacacctgta atcccagcta cttgggaggc tgaggcagaa 39660
gaatcacttg aatctgggag gcagaggttg cagtgagcca agatcgcgcc actgcactcc 39720
agcctgagtg acagagcgag actctgtctc aaaaaaaaaa acaaaaaatc tgttcttgcc 39780
aggcacggtg gctcatgctt ttaatcccag caactcagac ggccaaggca ggaggatcac 39840
ttgagcccag gagttgaggc tgcagtgagc tatgatcatg ccaccacact ccagcctagg 39900
caacagactg agaccccaac tcaaaaaaaa taaaaaacct tcctttctca cagaaaacaa 39960
acttccggat gggtattttt tttcttgggt gttttaaaac gataaattat ttagaagagg 40020
gaaacagggt aagcacactt gctgcttgtg ggtggaagtt ctgcaggaga ggctggcaag 40080
agctgatgca tgcaggttca cagtggtatc cgggctccat gttggcgccc atagctgtgc 40140
ctgctggctt ttctgcccac ttcagacaga ggtcccaaac cactcccact cccaacacga 40200
ctctcacatc tgctcactac caaattctct catttccagg tgtcagaaaa aaagcagcca 40260
tacctgtcct ggtctcactg tgccaatgca tttcataaca tctcacccaa agtctgcctt 40320
ctccacttac atgctgaaac tcggggccaa gtgtccatga gccactccct gctcttcctg 40380
taccagccta gcagtcctgg acaagtcact ttcttcccca agaccttttc tccaatctgt 40440
cccaccagga tcttctgaag catgaggcaa agccgtgctg acctccctct tcctcagacg 40500
tgcccagtcc aagctcattc ctacacaaga gccctcgtat ctactgttcc ctgtgcctgg 40560
aaagctggtc ccgatcctgt gtctgatcct tttcctaggt ctcggctcgg acaggcatct 40620
gggaccctct tcaggggggt tgtttcagcc cacctgactt acagcatccc cctacctggg 40680
tcccactgta acatatcatc ttgttctgtt ttcctcacag tacttatcac ttctcacttc 40740
ttgaaattat ttttttgttt tctttctttt tttttctttt ttcttttttc tttttttttt 40800
ttttttgaaa cagagtctcg ctctgtcacc catgctggaa ggcagtagtg tgattacagg 40860
tcactgcagc ctccaactcc tgggctcaag tgatcctcat gcctcagcct ccagcctccc 40920
aagtagctgg gaccataggc acatgccacc atacctggct aatttttgta ctttttgtag 40980
agacagggtt tccacatgtt acccaagctg gtctcgaatc cctgagctca agcaatcagc 41040
ctcccaaagt gctgggatta caggaatgag cctctttccc tggcctccct tcatttttaa 41100
taaaaattgt atgcaccaac ttaaggtgtc tcctgccctc tggctctgcc cacatctcag 41160
tccctccttt cctctacgtc cctgccagtc ttatctcctc ttgacctttc cgtccctcag 41220
caacttctac tatttccttc tgccccaaat gcctagaacc accactccct ctctgcccag 41280
caaactccct cttccctcct ttcaaagtcc tccagcctca cctcttccct gacgcctgcc 41340
cttgctcaga acacctccac gcaggtgaca gatcacccag tccaggatca ggcagtcgcc 41400
tgctctcctg agcagcccat gttcctcctc tgcttttccc tggccggcgg ggcctacagt 41460

ctgtccattt cacatgatca gcccctgggg gtcaggaatt ctgccttgca ctggcttcat 41520
tatgaaggtc atgccctgta ttcctgcctt ggcaacggaa atgctaggtg aatggtgggt 41580
atgtggatga aacgcacaag gctcatgcca ttaaggacac cttctggccg ggtcccttct 41640
tccctctctc agccacctcc tcctgcttgc taagcttccc caagaagaca gacccaggcc 41700
cgctcacctg agagccagaa gaggagcagg tagagtgtca gcaggggcat cttctcttca 41760
gacaggtccc cgttcccctc agtggagcct ggcagcagga acaaactaca gactcccagc 41820
cttctgctgg tactcactct cgcccttgaa cttcctcctt cagtcacttc ccatgggtga 41880
aagagactaa aagaggaagg aggtggttgg ttgggcaccg tggctcaggc ctgtaatcct 41940
ggcactttgg gaggccaagg cgggcggatc acgagatcag gagttcaaga tcagcctggc 42000
caacatagtg aaaccccgtc tctactagaa atacaaaaat tagccaggcg tggtggcgca 42060
cacctgtagt cccagctact caggaggctg agacagaaga atcgcttgaa cctagtaggc 42120
ggaggttgta gtgagccgag atcgtgccac ttgcactcca gcctgggtga cagaacaaga 42180
ctctgtctca aacaaaaaca aaaaaaaaaa aaaaaagagg aaggaggtgg aaagggtgaa 42240
atttcgaggc agttgacaat gcaaggggtc acaggaatca tcagcttctg agaagtacct 42300
ctctgtacac agccaccgtg ggttctctaa aaagagaact tcgtggctcc tctgtcatgt 42360
ggaaacctgc agacagatcc actgggtctg gccagccctt atccaatcct ccaaacctgg 42420
tgggttggct gggggcaggg gcacgcggct ccaccagagg ggtcctggag agccacggct 42480
gatagacacg atgtcctaaa cctggagctt caggtgtagc cagatgcttg ggagcccaga 42540
aggccagaag ttcaagagga ttgaggggca cttgtggcca aagtcagaaa gccagaggag 42600
agattccaga ccaaaggttg gaaggcagct atgaggagat tgggtatcca accccagact 42660
acaaggccag gtacggtggc tcacacctgt aatctcagca ctttaggagg ctgaggcagg 42720
aagatcactt gaggccagga gttcaagacc agcctgggca acatggcaag gcttcatctc 42780
cacaaaaaaa aaaaaaaaaa aaaaaaaaaa aagtcaggca tggtggctca tgcctgtaat 42840
cccaacactt tgggaggcca aggtaggcag atcactccag gtcaggagtt agagaccggc 42900
ctggccaaaa tggtgaaacc ccatctctac taaaaaatac aaaaattagc tgggcgtggt 42960
agcattcacc tgtaatccca gtgactcggg aggccgaggt aggagaatca cttgaaccca 43020
ggcggcagag gttgcagtga acccagatcg caccactgaa ctccaaccta ggcaacagag 43080
caagactctg tctcaaaaat aataacaata ataataataa ttagttctgc agagtcatgc 43140
atgcctgtag tccaagcaat ctgggaagct aaggcctgag gattacctga gcccaagagg 43200
tcgaaactgc agtgagctat gattgagcca ctgttcactg tactccagcc tgggcaacag 43260
agcagacgct gtctctttaa acacacacac acacacacac acacacacac acacacacgc 43320
acgcacagag aggttcagag agaagggaga tgcaagcagg gaactactgg cccactgaca 43380
agaggcgctt tgtcctgaga acacgaagga agctcctact gctgtacccc agcccaccag 43440
gtgagatccc agggatacct acatctggaa tcttccagaa acctgtccat tgtaccaggg 43500
ggtctacctg aggaaaccag ttcattgctc aggactatta tcccctcgct ccacccctca 43560

ccttataact atttgttgaa ggaaggaagg aagaaaggaa ggaccagttt tgcccctcac 43620
tctcctcctt ctgacaatca tgtccatccc ccatctctcc tttcccatca agaccccagc 43680
aagtccctag ggtcgggact ggggatataa aaggattagg aagggccaga ctcagtggct 43740
cacacttgta atcccagcat tttgggaggc caaggcaggt ggatcacctg aggtcaggag 43800
ttcgagatca gcctggccaa catggcaaaa ccccgtctct actaaaaata caaaacttag 43860
ccgggtgtgg tggtgggtgc ttataatccc agctactcag gaggctgaag caggagaata 43920
acttgaacac aggaggtgga gattgcagtg agctgagatc gcaccattgc actacagcct 43980
gggcaacaag agtgaaactc catctcaaaa taaataaata aataaaaata aataaaagga 44040
ttagaccttt ctcaccttct gcacctggag ccacttttgc catgtgtcac atacaccctg 44100
tgacatcttg acaagggagt gagtctcaaa gagggacgca accccctcac accctaatca 44160
ggaatgagtg ggtgtgcaga gggttatttg cacagaaaac cacagcagct gataagccat 44220
ggcttttcca ggacgtggcc agagatggct tctcgcttta catatctgca gaccataaag 44280
taggtcaaga atccggggtg agggcttggc cagccctttg gtccctgctg catgtcaata 44340
ggtcctctgc ccagggcacc atgcagatac tcagagagca atcagccctc tgcagtaggg 44400
aagccattgg cttccaggaa aaaggaccag gaatgggggc tgccctgaaa aacaaggggc 44460
agatgcatcg agggtggtgg ggtaagcttt cttctcctgg gcatgagcaa caaggaggga 44520
acagcactgt ctgttagaac tgcctgtggc cagaccctct gatcgtggcc tctcccagac 44580
ctggcctccc caagaaggat tcccctttga aacaagaaca gagaattcag gccgggcacg 44640
gtggcttaca cctgtgatct caactctttg ggaggctgag gcaggcggat cacctgaggt 44700
caggagttca ggaccagcct ggccaacatg tcgaaacccc atctctacta aaaaatacaa 44760
aaaattagcc gggcatggtg gtggatgtct gtaattctag ctactaagga ggctgaggca 44820
agagaattgc ttgaacccgg gaggcagagg ttgcagtaag ccgaggtcac aacactgcac 44880
tcctccagcc tgggtaactg agcaacacac tgtctcaaaa agaaaaagca gagaattcgc 44940
caagtggttc tgagtagctg ctcctctaac caagtgccta tggggaatac aggggaatcg 45000
gctcttctgc aggccttgcg tcctcaaaaa ctcatttgaa cctgtaatcc tggtttgacc 45060
attgacctat acacaagcca gttatatccc tgggcttccc tgtccccata tgtgaaagca 45120
gggattagac aggtcatctg agattctccc tgctctaacg cacctatgta tgtatgtatg 45180
tgtgtgtgtg catgtatgta cgtgaatggt tgaacctgtc attctgccct ggccttgtgt 45240
gctctgctag gggactctgt ctagcaccta agaaccagtc tctgttttta taaatcataa 45300
ttcttctcag aaaccattcc tgagcccttt tagttcccct cacttccaat tgcactttat 45360
ctttcccaac cacttgtgct atgtttcata ttttccatca ttattacatt gaatcctcac 45420
tataaatcta gaggaaaggt gatgcgtttc tcatttttca aagaaattga gacgcagaga 45480
gcaagtgacg tgtccaaggt cacatagcta gtaagtggca gagccaagat tcaaacccag 45540
gcctggttca tataagccat gctgccttgt gtcagggtca gggagggggc agagaaagag 45600

agagagagag agagagagaa aacagccctt agatcctgct cagtgctgac aatgaagaat 45660
tgataaagca gagaatcccg gagctggttg tcaccagggc gtccactcca aggttgtttt 45720
ttaaacaaat ttcctggccg ggtgcagtgg ctcacgcctg taatcccagc cctttgggag 45780
ggcaaggtag gcggatcacc tgaggtcagg agttcaagac cagcctgacc aacatggtga 45840
aaccctgtct ctactaaaaa tacaaaaaat tagcttggca tggtgatagg tacctgtagt 45900
cccagctgct caggaggctg aggcaggaga atcacttgaa cctgggaggc agaggttgca 45960
gtgagctgag atcgcaccat tgcattccag cctgggcaac gagagcgaaa ctctgtctca 46020
aaaataaaat aaaatgaaat aaaacaaatt tcagaatgaa agaatgtttt atcatgcact 46080
gaaaactgac ttattaagca ggtaactgat accaccagcc ttgaacattt tccagatctc 46140
atgcccctct tctgcagtgt atggaatggg aatctgggcc ttacccaagt tcttgtccag 46200
gaattcaggc acaggcaaag cttatttctg agaaaaatag gcgtctccaa agagtgagtc 46260
tgaaagtttg tgtctctcga atttcatacg ttgaaactta accttcaatg tgatggcatt 46320
acgaggttga gcctttggag agtaactagg tcaggagagt aactaggtct cctgaatggg 46380
attagtgccc ttataagaaa ggccccagag ggctgggcgc ggtggctcat gcctgtaacc 46440
ccagcactta gggaggccga ggcaggcaga tcacctgagg tcgggaggga gttcaagacc 46500
agcctaacca acatagagaa accccgtctc tactgaaaat acgaaattag ccagatgtgg 46560
tgcatgcctg taatcccagc tactcaggag gctgaggcag aagaattgct tgaacctggg 46620
aggcagaggt tgtggtgagc caagatcgta ccattgcact ccagcctggg cagcaagagc 46680
gaaactccat ctaaaaaaaa aaaaaaaaaa acagaaaaga aaaagaaagg tgccagagag 46740
ctgccttgcc ccttctacca tgtgaggaca cagcaagaag gagtcctccc tgaggagtgg 46800
gtcctcacca gatcctgagc ctaccagtgc cttgaccttg gactctccag gctccaaaac 46860
tgtgagaaat acatttctgt ttataaacca cacaatcagc agggcatagt ggcccacacc 46920
tgtaatccca gcactttggg aggccaaggc aggcagatcc cttgagatca ggagctccag 46980
accagcctgg ccaacatggt gaaactccgt ctctaccaaa agatacaatt agctgggcat 47040
ggtggcacga ccctgtagtc ccagctactt cggaggctga ggcaggggaa tcacctgagc 47100
ccaggagacg gaggttgcag tgagccaaga tcgtgccatt gcactccagc ctaggtgaca 47160
gagtgagaca ctgtctctaa aacatcatat catatcatac atcatatcat atcatatcat 47220
atcatatcat atcatatcat atcatatcca cccagtctat ggtattttgt tatagcagcc 47280
gggactaaga tagtgggtta gagaaaaaaa aagggctctt tgattttcag aatagagcac 47340
ctgtcatctt tgctacctgc tccttgacaa agtggaagag ctgcaattga ggctccccct 47400
gcctagaaaa tttgatcttg tttttaagca cctattaatt ggctagatgt atttggaaaa 47460
aaaaacaagc gtttgttcag caacaggtag gctaaacttg ggcatcccct tagctttcat 47520
cagattcccc agtgcaatgc acctcctcaa cacacacaca gactttttcc cactacaaaa 47580
gtaatgtgtg tttgttagag aaaaacagtg aaatacagat gaacacaaag aatgcatctt 47640
aagtcactcc gaatcctgcc acacagaaat aactgctgtt aaatatatgt gtgtgtatct 47700

attaccaatc ttttgttctt ctgtagaaaa gcaatttatt tcaggttaca tgaaggatca 47760
ctcatgtatg agtttgcttt accaactgga ctactacctt tattagggca ttttaataca 47820
ggaaaaacat aaaaagtatg gcagtggagg gtgttgataa tggggaaggt tatacatctg 47880
tgggggaaaa gggtctgtgg aaaaatccct gtatcttctg cccagtgtta atgtgaacct 47940
agaaattctc taaaaattaa atcctatttt ataaaaaaga gagacatggc cgaaaatacc 48000
agctctgaaa tacctccagt taatactttc tttaaaaagg taaaatgcag gccaagtttg 48060
gtggctcaca cctttaatct cagcactttg ggaggccaag atgggtggat tacttgagcc 48120
caggagatcg agaccagcct gggaaacatg gtgaaacccc aactctgcaa aaaatacaaa 48180
aaaattagct gggcacagtg gcatgtgcct gtagtcccag ttacttggga ggctgaagtg 48240
ggaggatcac ccgagcccag agaggtcgag gctgcagtga ctcgtcatca tgagccacag 48300
cactccagac tgggcaacac agcaagaact catctcaaaa aaataaataa ataaaataaa 48360
ataatttgct cacaattacc tttctttttc ttttcctttt ttttcttttg agatgggttc 48420
tcactgtgtc accaaggcta gagtacagtg gtgcaattat agctcaatgc agctgtaatt 48480
ctggaaaatc tcctggactc aagtaatcct cctgcctcag cctccccaat agctgggaat 48540
acaggcatgc accaccaagc ccagcttatt gttttattta ttgtagaata gggtcttcct 48600
atgttttcaa gctggtctcg aactcctggg ctcaagtgat cctcctgcct ttggcctccc 48660
caagcactgg aattacaagt gtgagccact gtacctggcc aagcaaatta tttaaataaa 48720
ttttttaaaa catagaaaaa gattaaatga aaagtaaaag tcttatctcc atttcccacc 48780
taccagcccc agaacctcaa aggcaatcct cttggcctct attgcctccc tgagaaaagt 48840
taatcatact ccagcagata aacctaagta acatatacac attcatcata ctatatacat 48900
cactctgccc cctgcttctt ttgcttaata atgaactttc tcccaaacag tgagacagga 48960
gagttccttt gacccttttg caggtaggaa ctggagggca ccagctctgg gtccagtcgg 49020
ccatttaggc accagcaggg acgaacttta cttgctcgaa cccactgggt tcagcccctc 49080
gtgagaggga gcacacaggc aagtgggtgc tagggctggg acgagtactt ttgggctctg 49140
gccccacggc agcatgaagg ggtgtgttac aattaaggtt cttttaggcc aggcatggtg 49200
gctcacgact gtaatgccag cactttggga ggccgaggtg ggtggatcac ctgaggccag 49260
gagttcaaga ccaacgtggc caacatggcg aaaccccatc tctactgaaa aaaaaaaccc 49320
aaaatttggc agggtgtggt ggcgggtgcc tataatccca gctacttggg aggctgaggc 49380
aggagaattg cttgaaccca ggaggtggag gttgcagtga gccaagatcc caccattaca 49440
ctccagcctg tgtgacagac tgagcctctg tctcaaaaaa ataaaagaaa agaaaaacac 49500
aattaatgct cttttagcag ttgccatcca cagatggcta agtgttagcc agctcagcgg 49560
agagtcaagg tgacagcctt tttacaccct gccctcttgg tacagagtta agtgttaacc 49620
agctcagtgg agagccaggg tgacagcctt ttacaccctg ctctcttggt acccgggttc 49680
ttgtcaggtg tccaggaaga atcaggtcac atagacttga aggatgatga atgcggagat 49740

tttattaagc ggtggaagtg gctctcagtg gaaggggagc tggaaaaggg atggtgtggg 49800
aagaaggtga tctttccctg aagcctggct ggagataacc gggctcctct ctgaagccgt 49860
gccatctgaa gttaagccgt gtctatccgt atgctccgat gctcagttgc ttccactctt 49920
gacgttcagc tgcttgtctc ttctctcctt ctcagctaca ccactctgct cgtctgccaa 49980
gagcttttta tgggtacagg gctggggaca tggtgggcca gggtggtttt ggaaaaggca 50040
acattcaggc aggaaaacag gaatgcatgt tctcatttag ggccgcaggt ccaggcttga 50100
gggtggagcc atcgccagga cctcgccctc ttttacccag tatttccctg ctgcaacagg 50160
gacatacagt acatacagat cttcctcatc cttttactgt tttcacagta ctgcattgta 50220
tgtatattct agagtttact tcactagtct ctactgctga attatttgtt cttacaaaca 50280
ctacaatgag catccttata cacatatctt ggtttacttt tttttttttt ttttgagatg 50340
gagtcttgtt ctgtcaccca ggctgtagtg taatggtgtg atcttggctg actgtaacct 50400
ccacctccca ggttcaagtg attctcctgc ctcagcttcc cgagtagctg gcattacagg 50460
tgcacgtcac catgccctgc taatttttgt attttcagta gagacagggt ttccctatgt 50520
tggtcaggct ggtctcaaac tcccaacctc aggtgatccg cctcggcctc ccaaagtgct 50580
gggattacag gcatgagcca ccacgcctgg tcctcttggt ttatttttgc aaggaaagcc 50640
atagagttgc tgagtcaaag gaatttgcac ttgaaacctt gatagatatt gtcagttgct 50700
atccaaaatg gttgctcaat gtatactccc actaatagtg tatgtatgag tgcctgtttc 50760
cccaaatcta agccagtact gaccattacc aaacttttat caagccgcta tacattatat 50820
tgtcattgta tcggttaggg ttctccagag aaacagaaac ggtaagctat atatatggaa 50880
agaaagagag atttgtcagc caggcacagt ggctcacgcc tgtaatccca gcactttggg 50940
aggctgagga cggtggatca cgaggtcagg agatcgagac cacagtgaaa ccccatctct 51000
accaaaaata caaaaaatta gccgggcgtg gtggcgggcg cctgtagtcc cagatacttg 51060
ggaggctgag gcaggagaat ggcgtgaacc cgggaggcag agcttgcagt gagtcaagat 51120
cgtgcccctg caccctggcc tgggcgacag agcgagactc tgtctcaaaa aaaaaaaaaa 51180
aaagaaagag agagagattt gtccgagaaa ttggcttaca tgactggggg ctggcaagtc 51240
caaactctgt agggccagcc caaaggctgt aaattttcag gcgagagttg atgctacaaa 51300
cacgaggcag aatttctgct tcctcaggga aacctcagtt ttgctcttaa actctttcaa 51360
ttaatcagag gacactcacc cagattactg aagacttaaa gtcagctgct tggagatgtt 51420
aacaacatca acaaagtacc ttcacagcaa catctagatt agtgtttaat tgaataagta 51480
ggactatagt ccagccaagt tgacacataa aactaaccac atgtacacaa aaatatatca 51540
acctattcac atcctttgtt cacttttcta ttgaagtttt cttctgagag ctctccttat 51600
atgagggata ttaccatatg tcatatgtct cgaaaatatc ttccctgtac catttctcat 51660
tcgactttgt ttgtggaatc ttctgtcaac caaaacattt ttattgcagc aaatctatcg 51720
atcttttctt tatggcatct gaatttcctg tcttgttaag aaggtcactc agagccactt 51780
taggagccat ggtaaccatc ccatttatca ggttttctaa aagctgtccc tgaataataa 51840

agtcacagaa aagcagcaat agtgtactta gacagcatta gcctggctcc cattgagagg 51900
cctggataaa gtaggattta atgttatgcc ctgcatttat caagggtcga tacaacagct 51960
ttagaaaaag ctttgcgtct tccgcaaagt tgcaattgcc cattttgtta ttcaaggccc 52020
cttgcagaag gatccactta acagattata gcagtgtata acaggagaaa atactcgatg 52080
gaaaggactc aggcatacaa tgtcagacaa gtaccataca ccccacctgg ttttcccctt 52140
tgccttgatt gtcaggaaac ctataaccaa acacaatgct caaacgcagt aacaacctaa 52200
cctcactctg ggatggaaga tttgccccag cccaccccac ccctacacac acacccctat 52260
tctttgtata ttttttcagg caatttcttg aactgccttc tcgtacttgt gggtttgttt 52320
gaaaccacct gtccccattg gaagtgaatg aggcacaaat acagatataa acaaaatgac 52380
tcaagaaaga acaaagacgc ttgtctgaat ggtggcaaag ttgacccaga ggtttctgtc 52440
tgaaccacaa ttaactaact tttttttttt tttttttttt gagacggagt ctcactctgt 52500
cgcccaggct ggagtgcagt ggcgtggtct cggctcacca caacctctgc ctcctggatt 52560
caagtgattc tcgtgcctca gcctcctgag tagctgggac tacaggcgcg catttttgcc 52620
tggctaattt ttgtattttt agtagagacg gggtttcact atgttggcag gctggtctca 52680
aactcctgac ctcgtgatcc acttgccttg gcctctcaaa atgctgggat tacaggcatg 52740
agccaccgca cccggccgta acattttcat aaatgcgaag agaaacacaa agagggaagg 52800
catggtgaca aatctggacc caaagttgac attcagcttc tccaagcagt gaaactggga 52860
accaacttgg agaaatttaa ggaattttca gcagctgttg ggtggcgaga gaagctctgc 52920
tgggttatct gtgaaaccag aaaatctgag acaagtctca gttaatttag aaagtttatt 52980
ttgccaaggt tcaggacgtg cccatgacac agcctcagga agtcctgacg acatgtggcc 53040
aaggtggtca gggcacagct aggctttaca cattttaggg agacatgaga catcaatcaa 53100
gatatgttaa gaagtgcatt ggtttggtct ggaaaggcgg gacaactcag aagcaaaggc 53160
agaagactgg aaacatggag ggagcttcca gatcacagat agttgcattc ttttgagttt 53220
ccaatgagcc tttccaaagg aggcaatcag atatgcatct atctcagtga gcagatgggt 53280
gacttagatg aatagatggg aggtaggttt gccctaagca gtttgcagct tgagctttcc 53340
ttagtgattt tgggggccta agatattttc ctttcacata tctgtctcat gtgtccgtgt 53400
gaagagacca ccaaacaggc tttgtgtgag caacgtggct gtttatttca cctgggtgca 53460
ggcaggctga atccgaaaag agagtcagca aagagcagtg ggattatcat tggttcttac 53520
aggtttgggg ataggcagtg gagttaggag caatgttttg cgggcaggga tggatctcac 53580
aaagtacatt ctcaagggtg gggagaatag aaaagaacct tcttaagggt aggggagatg 53640
acaaagtaca ttgaccagtt agggtggggc agaaacaaat cacaatggtg gaatgtgatc 53700
agttaaggct attttcacat cttttgtggc tcttcagttg cttcaggcca tctggatgta 53760
tacgtgcagg tcacagggga tatgatggct tagcttgggc tcaaaggcct gacaatatct 53820
aaggtaatgg atttaggcag ctccatccct aacatttggg ggccagggct agaatacaaa 53880

tggaggccca cataccatgt gtctcagtat cttaacattc taaagcaagc tcaaaaagct 53940
acaaaatcaa gtgtgtctaa tcttccctcc tgaacaaaat accttcttac caacctggaa 54000
gccaggttag catgtaggat tctcagaagt tccacactaa aatgtggcag cctagggaca 54060
aacaacctcc tgtccccagc cccccaccca caagctcctc tccttctcct cccacccttg 54120
ggcaccccaa ccccaaagga attttcagca gctgttgagt ggcgagagaa gacatgtaac 54180
aagtgctttt ggtttggtca gaagaatgca tgaggctcca catccaagct ctgtccacag 54240
cccctgcaaa tcgtcacccc ttggccacac cacagaccta gggatgccac cataagagca 54300
accttgggga aaagtgcagt cagtctacag gaggaatagg agcctttggc catgtggaca 54360
cctcgcttca tggggaggag cacagagaag gagcaggcct aagggaggtc ctaggtttag 54420
gaagagatca ttcaacaatg cttgtaaata gaccagcgct gagctctcca cgatggccta 54480
ggtaaaaagc tcttagagtc attatcagta gatttttttt tttttgagac ggagttttgc 54540
tctgtcaccc aggctagagt gcagtggcat gatcttggct caccgcaacc tccaccaccc 54600
gggttcaagg gattctcctg cctcagcctc ccaagtagct gggattacag gtgcctgcca 54660
tcacgcctgg ctaatttttg tatttttagt agagacagca tgttggccag gctgacttca 54720
aactcctgac ttcaggtgat ctgcccatct tggcctccca aagtgctggg attacaggct 54780
tgagccaccg cacctggcca ttgtcaatag atttctaatg ctgataaaaa aaagacctag 54840
cagggcattg tggcttacac ctgtaatccc agcactttgg gaggccgagg caggtggatc 54900
acgaggtcag gagatcgaga ccatcctggc taacacagtg aaaccctgtc tctactaaaa 54960
atacaaaaat tagccaggtg tggtggcggg cgcctgtagt cccagctact tgggaggctg 55020
aggcagaaga atagcttgaa ctcgggaggc aaaggttatt gtgagccgag attgtgacac 55080
cgcattccag cctgggcgac agagagagac tctgcctaaa aaaaaaaaaa aaaaagacca 55140
tgaccaatat gagaggatca tcctgcccca ccccgagcca aggttcagct gaatctgaaa 55200
tgctgaaccc tatgtgcacc cccggacctc agtgagctgg aaaatgtccc gagaagacag 55260
gactgtttcc tatgatgaaa attgatggaa actcctcacc ctggaaagga aaaggataaa 55320
agtgaaaaga attagaatct gtcacattct ttaagacacg aatgagggga acgtggatgg 55380
actacccctc acattctgga atattctctc taggaagcac ttcttaaagc ctgaggaagt 55440
tagattctag atgtaaatta ttaaaaccac tgcacataac aaacaaatga gttctctaac 55500
caggaggaaa taaaagctgg agaggttcag gaggtgggtg ggggcacagt catgggacat 55560
ccaaagtcaa tgccatggac aacctcatgg taagcagacg ctgatatgca ttcccttcca 55620
tgtcactgcc agagttgaac agtggctggg cccgacccaa agaggcccag ccaatctcta 55680
tgcttttacg gtttagttgt ttttatgtct ttatgttttt acaagtccag ctaaagtttg 55740
cacctcctag tcctttcaca cagtcttgaa ttcttgtctt tggaaagagg ccactgtcca 55800
ctttacacta aggcatttat ccactcaccc attcattcat ccattcattg agggtaccag 55860
cctctgggtg catgtagagt tcttctctgt tgtttatgat tttctttcaa acatttcatc 55920
tgaacttcca agctaattat cgccccttct ccctcccagt tcagacctcc cagaggaagg 55980

cggccttccc cctcttttcc cagaatctgc ttctctttga aatcttgtct tctccttgtt 56040
ctaagtccaa gtgtgcaagg cacataccag ctatctgatt ttgttttatt ctatagactg 56100
gatttttggg gctctcccac acaggagatc tatggacatt gtgttagtcc cggtcttccg 56160
aagaggagat gtgaagaggg aatcaaatgt gcaagggttt tactgaggga aatgcctgtg 56220
agagaaatag agtaggtgcc agggaggcca ggggagcctt cagaccatga tgcaggttgg 56280
atccccgtga aggaaagagg gcagaaaggt tgcatggaag catcccagac caccttgcag 56340
tatctggagg gttgtgcaag gccatgaggg agcccttgag ctaactcaac cgttagaggg 56400
gtcctgtgtc tcccaggaac aggcctgcct cagtgtccct gctgtgctcc gcctttggct 56460
ggtaattgcc tgtaggaagc atgaccttgg tgcaaacatg acaatgatgg ctttcagagc 56520
acggcagtgg ggaccatcgg tcatggatgc ccctgtggat ggaagtctga gagacacatt 56580
ctcatggtca cttcagatgc tcattaactt ggagggagat ctcctctgtg ccttcatgcc 56640
cctttcagtg acttcaatag aaaatcgaga ctttagtgtg tttgtgtgtc tgggtgtatg 56700
tgtgggtatc tgtgtgcatg tgtatgtgtt tttatgtgtg tgtgtctgtg tctgtgtttg 56760
tgtttatgtt tgtgtgtata tctgtgcatg tgtgcatgtt tgcgtgtatg ctgcatgtct 56820
gtgaatgtgt gtgtctgtgt gtcggtgcct gtgtatatgt gtctgtgtgt ctgtgtgaat 56880
gtgcatatgt atgtgtatat atatatgtgt ttgtgttata catgtctctg tgtatgcatg 56940
tctgtatatg catgtctatg tgtttctgag tttctgtgtg tgtgcatgtg tgtctgtgtg 57000
catgtctttg tatatgtttc tgtgtgcatg tgtttgtgtg cacatgtgtc tatgtctgtg 57060
tatatgcatg tctgtgcgaa tgtgtgtttc tgtgtgtttg catgtgtgct tgtgtctgta 57120
tatgaggata catgttggtg tacatgtgtt tccacatgtc tgtgtgcatg tgtgtgttct 57180
gtgtgtatgt gtgtgtttgt gtgtacatgt gtgttttatg tgtactatgt gtatttgtgt 57240
ttgtgtgtgt agatgttttt gtgtgtatgt gtatctgtgt gcatgtgtgt gttctttcta 57300
tgtgtgcatg tatgttttgt gtgtttgtgt tgtacaagtg tgtctgtgtc tgtatacgtg 57360
tgtttctgtg tgtacacgtt tttgtgtctg tgtgtgcatg tgtgtttctc tgtgtgtgca 57420
tgtgtttgtg tgtgtgccta tgtttctgca tcatgtgtgt tctgtgcata tgtgttgtat 57480
gtgtgtatgt ttatgtgtgt gtgcctctgt atgcatctgt ttctgtatgt atatgtgtgc 57540
tctgtgtatg catatgtttg tgtgtgcatg tgtgttctgt gcatgtgtgt gcatgtgtgt 57600
tctgtgcata tatgtgtaca tgtgtttttc tgtgtctgta tgtgtcttgt gcatgtatgt 57660
ttctgtgtca tgtgtgttct gtgcatacat gtgtacatgt gtttttctgt gtctgtatgt 57720
gtcttgtgca tgtatgtttc tgtgccatgt gtgttctgtg tgtatgtgtg tacatgtgtt 57780
tttctgtgtc tgtacgtgtc ttgtgcatgt atgtttctgt gtcgtgtgtg ttctgtgtgt 57840
atgtgttgta catgtgtgta cgtgtatgtg tgtgcttctg tgtgtatgtt tctgtgtgta 57900
tgtgtgttct gtgtgtgcat atatgtttgt gtgtgcatgg gtgttctgtg tatgtgtgta 57960
catgtttttc tatgtctgta tgtgtgtgtc agtgtcttgt gtgtgtgtgt ttctgtgtgt 58020

gtgtgtgcat gttgtgcaca tgcgtgctta gaaagaatcc actcccacag ccacaaggac 58080
acctctgagg ccaactcaca tcacaggccc tcagctctct ctctattgtt cccttccaga 58140
ccatcctggt gggtgacagt ggtgtgggaa agacgtctct gctggttcag ttcgatcagg 58200
gcaagttcat ccccggctcc ttctcggcca ctgtgggcat cggattcacg gtaagcactg 58260
gccggcactg ccagctctgg gcctgggctc aggaccccag cgtgtttctg ttgagtgcca 58320
gcaggaaaca ggtggacact cgcattggat cattcaagga ggattaagga gaagactgtt 58380
ccccaaggtg taggagggtg ttgggtgacc aggagggagg gtatactgcc cctgggtagt 58440
ccaggaactc cgcccacctc tagcctcaag caggaaggga aggcaccctc accagaaccc 58500
agggacaaag caggctgctg ggtgagctgc ctggcaggag ctgcgtggga ctttcagtgg 58560
agggacacag cagcccatgg gagcccccca gggaggcagc caggagaaca atcccctgtc 58620
ccaccttgct ctcctcctgc tccccatctc cttctggggc tccacattgg atgaacgcaa 58680
gtggaagcca gaaagcaaaa aatattttaa tatgggatag ctcaggtcag ccctccaagg 58740
caagaagatc tggagggaca aagggacgac aaagttttca gcacagtcca aacttagcag 58800
tcacacgggt ctcagggagc tggcaggccc aggttacagt tccgtgttta gtcctcggta 58860
aaatggtaaa agggcagctc gggttaagga agcccagtgc cctcggcttt ttctgcccgc 58920
agcccctctg cgagacaagg gatccctcct ttaccacaca tcccatctcc ccgcgttgtc 58980
ccgtgcctgc ggctgcagct gcccttgaat tcctgggaag accttgggag agggttccac 59040
tcctctctcg ggctgtggca ggaagagcag ggccttccct tgggactcca gaatcctagc 59100
atctcaagat ggggaagagc aaacatctct tcttggacaa cgcggatgtt tgggaacctc 59160
tgagaccaga gcacagggcc tgtgagatca cgctcagggt caggacgcag cagtgccaca 59220
ctgatgggaa gagagagagg aaaactcaga aaaatgggct tgcccactgg gtcacagcca 59280
ggaaggcagg ggttcagaat gaacatggct tctgcatgac cttccaggag gcaaagcgga 59340
agtgagtgcc tggttcgggt gtgttctggg gcctagaatc ggccctgaaa caccagttcc 59400
cagggctctc cccagactgg gaccggctgg gctctggtag tgctgaatag ggaagggctg 59460
agacccagga ggccaaggct acctggcaat ccctgaggtc tgggaccagg ctgccatgaa 59520
gcaaaccagg atgacctggt gccaggcgtc acccacccca gcccaggccc agtctgctct 59580
cagcagccag ggcacaccct gccctcaggg tcacacccgc atctcctcac ccacccttct 59640
gcctgtctgg tgaggaagga agagagtggg gtggatgtgg ctgtggacat gcaaagtctg 59700
actcacgttc cacagaccac agaggtgaga tctccaaatt ttgccgctag cttggcaaga 59760
aaattgaggc cgatttcctg tgaaggaaaa agaaagtgtg tggtcaaact gtttgtctag 59820
cctgagatga gacacactgg gctttcccac tgacctcagg gtgggcctcc tagagcctgg 59880
gctgcctgct tggtaccagc cagcacccga tgttctggtg catctcagga caaacctgtt 59940
gcctgcttac gctgagtgag gacctgagcc cctggccaga aactcagcct ggggacacct 60000
gagcaaagaa ctggtggaca tcggggtctc ctccagatat cagcggtctg ggggtccccg 60060
gagagccagg ctacaagaca cttgtttacc catctgtcct tctcacggag atttgagata 60120

tggcatggat aagggccctg ccgctggcca ggctgtagcc tgcaactcct gcatgatggg 60180
aagggaggca ggtgctgcca caggctccgc tcaggaggct gagtgccgcc caggtggcag 60240
gggagggaca gagcccctgg tctcccactt tggaggagcc agaggtgagc agggtgacta 60300
cctcgggcat gccttcaccc ataaactacc aggcccatcg ccttctggaa gtgggacctg 60360
cccaggtcct gtgtagatgc ctctcagtca agaccttccc taaattctcc actaccagcc 60420
ccatcaaact ggctgtgggg gtcgtgctga tggggttatg gcacactgat gtcgatgggg 60480
ttcacacagg ggtctttcca gtccaccaca gttcaggtcc tccttgttac ctctctcctc 60540
agtttccccc aaattcccta gtcttgccta aacactgatg caaaattcct ctttcaaaac 60600
cttcactcag gccaggcgtg gtggctcaca cctgtaatcc cagcactttg ggaggccgag 60660
gcgggcggat cacttgaggc caggagttca agaccagcct ggccaacatg gcaaaacccc 60720
ttctctacta aaaatacaaa aacgtttgcc aggcgtggtg gcacacgcct gtaatcccag 60780
ctacttggga ggctgaggca agagaatcac ttgaacctgg gaggcggagg ttgcggcgag 60840
ccaatatcat gccattgcgc tacagcctgg gcaacagagt gagactccat ctcaaaacaa 60900
caacaacaaa aaaaaaaaaa cacctcactc tgctcatccc ccacctgaac attcacggtt 60960
cactattacc ttccaaataa agtctgaatc tctcatctgt tggtcaagct gttgcacagt 61020
ttagaccagt ggttttcgaa cactccttca gcgcacataa aagcctccag ggggaagctc 61080
atctgaaatg caaattctaa gctccctcat acacatgctg cttcagtaaa accacgtggg 61140
gggcccagaa tctgggggtt ctgacccagg gggtccatgc gccacatttt gcaaaacatg 61200
agttgaggcc ttgtccctcc agtctctgcc cgggtctcct tcccacatcc cgaccctgtg 61260
ctctttgggc tgtgctgttc cccagctctg aagttttctg ccatcgacct ggatagcagg 61320
gagtcccctg cccccatctc tgcaggtgtg atttgaggtg tgtgtggtga ggtgatttga 61380
ggggtgtggc aggtgtgtgg tgtgatttga ggtgcgtggt gaggtgtgtg gtgtgatttg 61440
aggggttagg tgtgtgtggt gtgagtgtgt gtgtatggtg aggtgtgtgt gtctggggtg 61500
tgatttgaag tgtgtgtggt gtgaggtgta tgtggtgtga tttgatgtgt gtgtatgtgg 61560
cgtgagatgt gtatggtgtg atttgagggg tgtgtggtgt gacgtgtgtg gtgtgatttg 61620
aggtgtgtgt gatgaggtgt gtggtgtgat ttgaggggtg aggtgtgtgt ggtgtgaggt 61680
gtgtgtggcg tgatttgagg tgtgtgtgtg tggtttcagg tgtacacaag agccccccca 61740
tctttcaaca cgcatgaggg tctgtctcac tgacacttgg tacctccact atcagcaggg 61800
ctgcatgaca atgacagtgt gatctgtgat caggagggta ggggtgtcta tttgggggta 61860
ttgtgggggt gtgcgtctca gatctggagg caataagagg tgttgggggt catttatagg 61920
gtgatataca catttggtga tggtgtgagg catggggtcc attggagagg caggtgtttg 61980
cacagtgatg tggcacaggt gttgatggtg tgaaataaac tgtgcctgga gctgcactgt 62040
ggtgacagga gtgtgtgttt ggagtgaaat gaggtatgtg tgtttgaggg tcatgtaaga 62100
tgtgtatgta tgtggtatga tggtaggtgt gtttgtttat agtatgaggt gcacatttgt 62160

ggcataatac aaagttgtgt gtgggacatg ttatgaagtg tgtgtttgtg gtataatgta 62220
gggtgtgagg catgtgtatt tatggtatga tgtgaggcag atgtgtgtgg tatgatgtag 62280
ggtgtgtgtg tatatttgtg gtgtgatttg agagtgtatg tgtgtgatgt gaggtgtgtg 62340
tgatttgagg gatgtgtgtg gtgtgaggtg tgtggtgtga tttgaggtgt gtgtggtgag 62400
gtgtgtggtg tgatttgagg ggtgaggtgt gtatggtctg aggtgtgtgt gtgtggtgag 62460
gtgtgtgtgt ctgtggtgtg ttgtgggtgg gtgcacacgt gtgagtgtct gtggtgtgtg 62520
tctggggaga gacacaggct accacagcgc atacacagcc tcagagtgaa cgttaggagg 62580
cccaggtttc ctccccagag accacacgga tcccttagtg agtccctatt ctgcacagcc 62640
cctgttccct gttctgaatg tcttagaccc taacccactt ctgactcacc cgaggctgcc 62700
cttgaacacc ctacaggaca ttacagtggc cctggggaca gtgtacgcac cctccaagta 62760
tgacattacc cacataccac agacccagca ggcctgcagc ccctctcccc atgttcaaag 62820
acttcttccc taggtcagat tcctccatcc agactagtgc agcaacgcag cagggaagtg 62880
cttcaggcca ctgctccttc tggatgattc gtgctaatgt tcaaccttct gtattccaaa 62940
cccaccatag gacaaagagg gccttggagc caacaacgtg aaccacagct ggaggctaga 63000
aatctgaact caaagtacca gcagggccac gctccctctg aaggctccag agaagggtca 63060
ttccttcctc ttccatggcc ttggtggctc ccaggagtcc ttggcattcc ttggcttgca 63120
gccacactcc tcccatcttg cctctgtctt cacctggatg tcttttccct gtgtctcttt 63180
cttcttatga caccggtcat actggattaa tggcctgccc tatattagta taacctcatc 63240
ttaatgagtg atatctgcaa tgaccacatt tccaagtaag ggcatgatct gaggtactgg 63300
tggttcagac ctcaacctgt ctttggggga cagaattcaa cccataacag catggtttac 63360
agtagaaggt gagcatgagg catgccctgt cccctctctc attcttcctc caccatttcc 63420
ctcctgcaat gaaaacccgc atcctggctg ggcacagtgg ttcacacctg taatcccaat 63480
actttgggag gctaaggcag gcggatcacc taaggtcagg agttcaagac cagcctggcc 63540
aacatggtga aaccctgtct ctacaaaaat acaaaaatta gccaggcatg atgatgggca 63600
cctgttatcc cagctacttg ggaggctgag gcaggaggag aatggcttga acagcccggg 63660
aggcggaggt tgcagtgagc caagatcgtg ccacttactc cagcctggat gacagagctc 63720
cacctcaaga aagaaacaaa aagaaagaaa gaaagagaga cagagagaaa gaaagggaaa 63780
gagagagagg aaggaaggaa ggaaggagag aaagaaagag aaagaaagaa agagagaaag 63840
aagaaagaaa gaaaaagaaa gaaagaaaaa gaaaggaaga aagaaaaaga aaggaaggaa 63900
ggaagaaaga aagaaagaaa gaaagaaaga aagaaagaaa gaaagagaaa gaaagggaaa 63960
agaaggaagg aaaagagagg ggaaggaagg aaggcaggca ggcaaagaaa gaaagaagag 64020
agagagagag aaaggaaaga aggaaagaag gaaggaaggg agggagggag gaaggaagga 64080
agggagggag gaagggagga gaggggaggg gagggaaggg gagagaaaag accgcatacc 64140
aggccaggtg cggtggctca cgcttgtaat cccagcaatt tggaaggcca aggcaggcgt 64200
atcgcctgag gtcagcagtt ccaaaccagc ctgtccaaca tggtgaagtt ctctactaag 64260

aatacaaaaa ttagccaggc gtggtggcgt gcacctgtag tcccagctgc tccagaggct 64320
gaggcaggag aattgcttga acctgggagg cagaggctgc aatgcgccaa gatcccgcca 64380
ctgcactcca gcctgggcga cagagtgaga ctccgtctcc gggagcccac ggcattgagc 64440
aaacctcggc attatttgca gcaagagcct ctggcatcca aatagcaacc aacaccacgc 64500
ctctgtagtg tgctgcgcag cctccacact ccagtctgag gctccctgtt tgagtcccgc 64560
cctatgccca gctgaggtta tagcacgctc acctccagaa gaggtaaccc aagctcttta 64620
ctctactgga gatcacctct gtccccactc tgggcgcttc tcccagctga cagaaaatac 64680
ctccagctga tgtcagaaaa tacagggctg gaggctggcg tacaaagtca gtccccacag 64740
gcctatggtg gcccataagc cacgtctacc cctgctcctc acctccacac ctaagttaag 64800
aattgcaggc cgggcgcagt ggctcacgcc tgtaatccca gcactttggg aggctgaggt 64860
gggcggaccg cctgaggtca ggaatttgag accagcttgg ccaacatggc aaaaccccgt 64920
ctctactaaa aatacaaaaa gaaaaaatag ccgggcctga tgtcgcgcac ctgtaatccc 64980
agctactccg ggagactgag gcgggagtat agcttgaacc cgggaagcaa aggttgcagt 65040
gaggcgagat cgcaccactg cactccaggc tgggcgacag agtgagactc tgtctgaaaa 65100
aaaaaaaaag tgcaggtacc cctctccagc tctcccctcc ctacacatcc ctcaaaccgt 65160
cccgctgtaa tgcacccgcc ctgttccttg gtaacttgaa gctgcttata gaatgtggag 65220
atgggggtaa ttgaaaggtc ggcccaggcc acagagcccc tgagctctgc taccggcaac 65280
cccagctgca ctccccactc tctgtcacca ggagctgccg ggtgcctggg atatcctggc 65340
agctctgctc aaaatgatct acgacttcat gaatttattt ggctcctcct cggggccagg 65400
gtgagtgtca tgggttaata aggccggccc cgccttcagg agcggtccac tgggagatgt 65460
gtgctgcgca gccctcttgc gaaagctctc ccctggtggg acattctggg cacaaccaac 65520
aggccggggg aaatgagagg tgatccatac taaagggtca aagtccccgc accaggcaga 65580
ggccccaaaa caccgcagcg tacatgtgct gcaaggcgag tacgggttgg taaacaaaac 65640
tatattcaga tgagctcggg ccgggtgact taacagatga ggaagtgtct cggggccatc 65700
ggcggaggcg cagcccaggg gtccccagct ccccgcctcg ccacctgggg acagcccacg 65760
gcccggggct cggcggccgc ctgctgtcgc ggtgcgcagc gactacggga actcttccgc 65820
agcagacggg gtccccgcgg cccgctcccc caggggcaag caagcgacca caggggaccg 65880
gtcccggggc tggatgtggc tcatgtccga agcgcacgga gccgagccgg tgttgctcag 65940
ggaggctgcc cgccccttca cgcagaccct gcggctctgc gtgccctcag ggaacagcaa 66000
ggtccgagcc ggtgtcgtcg agggggcgac gggacggagg gaggagcctg aggggtcccg 66060
gtcgagggag gggaggagtg ggcggggcgg gggtggggcc gttcctgcgc tctccttcgc 66120
ctgcgggccg gcactgctca cctctcgtcc agggacatga cgggcacgcc aggcgccgtt 66180
gccacccggg atggcgaggc ccccgagcgc tccccgccct gcagtccgag ctacgacctc 66240
acgggcaagg tgggtgggcc tcttccgtga gacccccgcc ctcctcggcg ctagcccctt 66300

cctggctgcg tctgggttgg actcagccct tcccccaggc agctgcgtct cccagaggag 66360
ggagggagag agggtcagga cacagcctct ggggccgtcc caagctctag gtgtctctgc 66420
tggcttggtg ggggcgggtc gcggaagatc gcaaaaactg agtgatcccc ccgccggccc 66480
caactcagtt ctcttctgcc acactctggc aaatatgagc ccccgggagc ccatgcttct 66540
tggtgagggt taagcgcgca actctcgggg ctcaggctgg gaagggctgg gagatgggga 66600
ccgaacggag actcggagag gacgtcccct gctggcagag gaactggcgt taatgccatt 66660
ttccgagcta agctcttagt tgagatctga catccaggtt taaggcctga tgtcccccag 66720
ctgctcccct cccattccac ccgctggagg cactgcctcc caccttcctc cctgcagtcg 66780
gaagccgctc ctcccagaag gatgttgcca gccggcctgc aggtcacttg ggaatttttc 66840
gaacctgaga aagatttcag tggttggtct ttcgcatccc gcacttgaga gagctccagg 66900
gctgctctct ggggcttgct ccctctacag gggtgtcctg tatggaaaca ggtagggaca 66960
gcagtggact ggtctgtcgc cttccatctg tgtccttgga gtgagcgggt accagaaact 67020
gaaagaactg ctgagggagc ctagagcttc cactcttcct ctgcagggtt ggggatggag 67080
tgagggctgt cctggattcc gctgcatggc cttgaaggag acctgcctct ctctgggcct 67140
cggtttcctc cccgacacca gggctcaccc ttgctgggag cctcagcctc caccccagtg 67200
tttcggggga agccaccctg caagtcatcc gcccagagcc gttgagatag gcgtcctgtg 67260
tgggcttgtg gcaggaaatg ggcccctgca ccctcggaga ggaggagctg ctgttggcca 67320
ggccccaggc tgagggggac tgcctgacct tgttgccctg caaaccagct gggttgtttg 67380
cctaggaggt ggccaggcta ggcagctgtt tgtgtttggt ggaatcaccg agctgggtgg 67440
gtagctggca tcgtttgctc aaggcagctg tgatctgtaa agtacacaaa gactggccct 67500
ccctccctcc ttcctgctcc agggctggga cccaggagcc agggaggagt gcaggctcca 67560
gaaagctcct atcccccacc ccttcatctg ttccctggcc aagcggcatt ggccggagag 67620
ttggtcccca gcctccccgg gcctgcccca ggggagtgag tccaggaccc tctgagaaag 67680
cctggcagga gctccttgga ccagactagg ggtgatgtgg cccacaggca gacagttccc 67740
accctgggcc actcttccct gggtcttagg tgattcacca cgatgatggg ccctagccat 67800
taacagactc tagaaatacc tcaaagacat tatccctcct ccttctaccc actatggaaa 67860
ccatgccaca gaaaggttaa ggaatcttcc taaagtcaca cagtaggcca tttacaaatc 67920
aagacccatc cttcataccc cttctgctca gccacccctg cctctccacc agagttaact 67980
aatgccagta ccccatgccc acaacaggaa tgcctttggg ctccactgtc aatttcagag 68040
cctcaaaaat aattcaaacc tagtccctgc ttaacccatt aagccaccta accagcagct 68100
gggaaattcc agcattggat ctagacccct gttatccaag attggagaac agtgggacaa 68160
agtgctcctc tccaccattc ctgcgtgtcc ctggggaaga tgagcagagc agagccagac 68220
agtaaaggag agggccacgc cccctccaca ggttacctcc ttggtactcc tgcccgcact 68280
acccacagca accccgggat gccgatctgc agccacatgt cccatgtggg aggtttctgc 68340
tgaaagaact tccaactaca catctcccca cttcagtata aatttcaacc ttccctaatt 68400

catgcaacct tttttttttt tttttttttt tgagacagag tgtcgctctg tcaccgaggc 68460
tggagttcag tgatgcaatc tcggctcact gcaacctcta cctcctgggt tcaagctatt 68520
ctcctgtctc cgcctcccaa gtaactggga ctacaggcgt gtgccaccac tcctggctag 68580
ttttttgtat ttttagtaga gatggggttt caccttgttg gtcaggctgg tctcaaactc 68640
ccaacctcag gtgatccgtc caccttgggc acccaaaatg ctaggattac acacatgagc 68700
tactgcaccc ggcctcatgc aaccttttaa taaacataaa atgtcattag aaacttccag 68760
tcaggcgcgg tggctcacgc ccgtaatccc agcactttgg gaggctgagg cgggcggatc 68820
acctgaggtc gggagttcaa gaccagcctg gccaacatgg agaaaccccg tctctactaa 68880
aaataaaaaa ttagccaggc gaggtggtgc atgcctataa tcccagctac tcaggaggct 68940
gaggcaggag aatcatttaa acctgggagg tggaggttgt ggtgagccaa gatctcgcca 69000
ttgcactcca gcctgggcaa caagagcaaa actccatctc aaaaaaaaaa agaaagaaag 69060
aaagaaagaa acttccaaat aaatgttgtg acacaaaaaa aaaaacccaa acaatattca 69120
ttatagagta tgcaaatgac catgccccac ccccagcaga ttctgataga ctcccttggg 69180
tgggaatcct tgtccaatat attgacactt ccctttcctg tcagtatagc ccagcccatg 69240
cgtgtactca cgagcggacg atggatgaca caagtacaca gagggacgga atccctgcat 69300
ggtgtggcta tgggcaaatg tggccactgt ctagattgtg caaatgtggt ggttctctgg 69360
ggccacagag cacacttggg gacctgttca tggtgaggtc tcaactccgg cctctaggaa 69420
cttgaatgag gacaggaggg tcagagggag agcctaggag gctgagccaa ggagcgtgga 69480
gaggagagac agggtgaaga tggcggctgg ctttctggaa gcaggtggcc tttggtgcgg 69540
tcagcattcg tgccagcccc ctcttctctg atcctctcca tgtgtctctc tcctggaatc 69600
ccagaagctg cccctgactc cccattaact gcctctgccc ctacccccta ggtgatgctt 69660
ctgggagaca caggcgtcgg caaaacatgt ttcctgatcc aattcaaaga cggggccttc 69720
ctgtccggaa ccttcatagc caccgtcggc atagacttca gggtgaggtg gctgcaggca 69780
cttgcttcca gcagagagcc agggctgtgg ctcaggcatg ggggggttgc ccccaccttg 69840
ctcaccctgg ctcccaggga ctcccgaggc tcatgcctgg agggcacaca acccgctccc 69900
ccaagaccac agaggtggcc gggtcaaagg agactgggca aggttggctc cttgcccaac 69960
tataggatgc aaaaaaatga gactgagtct tcgattccag ctccattcct gggggacttc 70020
tcccaagcag agcagccgca ggcacggcat aagctgaata tcttggccca cagagcccct 70080
gctcattgct ctcctacctg ggcccctttg gaaaggcctc aaaggtcaat cagtctttct 70140
ggagttccca gaaagcacag ccctgcactg ggtttaagag ctgggcttgg gccgggcatg 70200
gtggctcttg cctgtattcc cagcactttg ggaggccgaa gcggtcagat cacaaggtca 70260
ggagtttgag accagcctgg ccaacatggt gaaaccccgt ctctactaaa aatacaaaaa 70320
ttagccaggt gtagtggcac gctcctgcag tcccagctac tcgggaggct gaggcaggag 70380
aatcgctcaa atccgggtgg tggaggttgc agtgagctga gatcgcgcca ctgcactcca 70440

gcctgggcaa caaagtgaga ctgcgtctca gaaaaaaaaa aaaaaaagag ctgggctggc 70500
catgttggga gacagcagct caccagggac cctccctctc accttgacga ctccatctta 70560
caaatctgca tcagggatgc tagacgctgc acacctgaag tgttcaatag agaaaaggtc 70620
tcaccctggc aggtggggct ctacagcttc aagcaggcag aaagcgaaca cttccttcac 70680
tagagaatta gtgggcagct aaagaaaagg tgctgctgca gatgtagcct caggtcccca 70740
ggatgcaggc aaacacccca tctccagggg ctcggtcaca gtcccaaggc taggctccag 70800
gagagggaga ccgaagtggg aaaagggcag ggcctccagc agcaaccagc cctccagccc 70860
tgggctgcct gatccctgga gagagccagg atgtttctca ggctcctctt gccctgctgt 70920
tgtgagaagg cagttacagt cctcagaagg gacgactcca cagtggaggt gtctgggtat 70980
ggggttcctg ctgccctgat ggtatgatct ggctggagac ggttctgggg ctcactgcac 71040
ccactctagg cctggagagg gaacaagaca ggacgtctgc agagctgagg agccacatga 71100
ctcctgccct cccatcctct gccttttctc tttcagaac aaggtggtga ctgtggatgg 71160
cgtgagagtg aagctgcagg tgagaccaga ggctggagtt ggggagggag gatggaggac 71220
ctgcccttcc ttctcaccct gaaccacagg aggcctgcag ccctgccctc cgcctggggc 71280
aatttcctgt ggggcccacg ggaggaaatg gcttttgttt atttgacatc tgcagaaaaa 71340
gcagttccca ggcaccctct catctatgaa cagcagctcc aaatgccttc agacaagctt 71400
agcctccatc catctcctcc ccagttgcca gggctttatc tgctcttagg agattggaca 71460
tccccaaccc ctgagctagg ggagaggaga agattctttt tttttctttt cttttctttt 71520
tttttttgag atggagtctc gctctgtcgc ccaggctgga gtgcagtggc acaatctcgg 71580
ctcactgcaa cctctgcctc ccaggtttaa gagattctcc tgcctcagcc tcctgagtag 71640
ctgagactac aggtgcatgc caccacacct ggctaatttt ttgtattttt agtagagacg 71700
gggtttcact gtgttagcca ggatggtctg gatctcctga cctcgtgatc cgcctgcctc 71760
ggcctcccaa agtgctggga ttacaggtgt aagccaccgc gctcggctga ggagatgatt 71820
ttgaacgagc ttgagaaatc agtaactgct actgtccagg tcattggatg ctcaggggct 71880
catgagaacc taaagaagaa aacagcccca ccttcccaca gatatctcat acaacaaagc 71940
aggcctgctc cacccagcac attccttgca cctgcctcct tctgaccatt tctccatccc 72000
atcccttccc agatctggga caccgctggg caggaacggt tccgaagcgt cacccatgct 72060
tattacagag atgctcaggg tgagtccctc gcaccctcca acccctaccc cagccccttg 72120
gtagcatccg tgctgctgcc taagtcccct ctgtgatcct ctcccctcca gccttgcttc 72180
tgctgtatga catcaccaac aaatcttctt tcgacaacat cagggtaggt cctcccttcc 72240
cctgactccc acccataagc agccaaggca aggtctatgc aggctggggt tgcttcctgc 72300
cctgtggaaa gcgggtggag cgtggagtcc tcctgccttc tgaaaaacac ctacttgtga 72360
ctcagaagtc atatctgctg ctttgtattt ggtggccatg tgggcatgaa ggccaagcag 72420
gctgttgtga ccctgtgcca cctgcatagc cctcactgtg attcacgagt gtgtttcgtg 72480
acaaagtgtt cagaacagcc cccactccac cctggataat tatccacaga gaccaaggga 72540

aaaacacaac cagaaaagtc cacacataca tccagggcaa gttgcaagaa agtgactcag 72600
tcagacagag tgagtggttg tatcctcaca accaaactat tatagagaca aaaatttgat 72660
aaattcaagc accaattttg ttcacgacat tgtataggtt tcatgaatcc cctgacctca 72720
aggacagttt gctgataagc aaactaggag aataaaacgt ttatatagaa agaggaaaat 72780
ccatggcact catactccta cctccaaccc catgctcatg gcagacatca ctaatcaatc 72840
acagtacttt tgatcactga aacccttatg tggtcttaga atctttaaca ggacactcca 72900
agaaatcact gctgacagcc aactgatttg tgagataagg tctccatgca tctggatctt 72960
ccatagaact gatagttgca cagcataaaa tggtgagggt ggggccattg tgggttgagc 73020
caccaaggaa ggccatccag gcctggatgg gccagaacaa aggtacagat gagagaacgc 73080
acagggtatc gtgttcaagg tagtgagtaa ctgaggatag tcaaacggag cagaagaaga 73140
aaggggcagc aggaggaaga gaatgccagt ctcgcacgcc ctctcccaca ggcctggctc 73200
actgagattc atgagtatgc ccagagggac gtggtgatca tgctgctagg caacaaggtg 73260
agtggctccg gggcagggtc agcccagccc tgcacttcct cagccctagc cggccccata 73320
accacccaag aacagttatc taggcatcct tcctgaaaag gactctgcag cctccagctc 73380
aggggtcaga catatctgga ggcttctgcc catcccatct gccccttcca gggaaagtcc 73440
aagttgttgc ctgagaaatc aaggggtgcc cagttctcag cccccattag agcagagtga 73500
acagggtccc aggtcagggg ctaagagtgc aaagggttag ccccaactgc tgtcctattc 73560
caagaccctt taccaaaggt gagatcccag agctgggagc tacactgggc agaaaccctg 73620
gccccaggcc aatcacacct gcctgcagtc ccttgggcca ccagcagagg gcaggcaacg 73680
cctgcttctg gggcaaaata tgggcccgct ggggcggagg cctccttccc cagagtgacc 73740
catttgggct tgacaggcgg atatgagcag cgaaagagtg atccgttccg aagacggaga 73800
gaccttggcc agggtaagtg attgtctgtg ggacagggtg aagggtgggg gcaacccgac 73860
gctggccctg aggacactct ctcccgggca ggagtacggt gttcccttcc tggagaccag 73920
cgccaagact ggcatgaatg tggagttagc ctttctggcc atcgccaagt gagagctggg 73980
cagggaaggg aagtgtgcgg ggcagggcgg cacactccag gaatccagta gggcccggcc 74040
cctggcccag cccctggaca cacctgcatt ctgcaggctg aggtccattt gctctgggag 74100
cactgggcca ctgggagagg ggagggggcg gctcagctcc tcaccccagc ccagcccagc 74160
ccagcccagc ccattgtctc ttcttcaagg gaactgaaat accgggccgg gcatcaggcg 74220
gatgagccca gcttccagat ccgagactat gtagagtccc agaagaagcg ctccagctgc 74280
tgctccttca tgtgaatccc agggggcaga gaggaggctc tggaggcaca caggatgcag 74340
ccttccccct cccaggcctg gcttattcca agaggctgag ccaatgggga gaaagatgga 74400
ggactcactg cacagccgct tcctagcagg gagctatact ccaactccta cttgagttcc 74460
tgcggtctcc ccgcatccac agggagggta aaacacttag cttttatttt aatagtacat 74520
aatttaatac caaaaaaggc gcctggatcc ccaaaaaacc gaggctggga gctagtggcc 74580

cttttgcttt ctaggacttg gggggccggc cctccctcct aagcataaca aaggtggtgt 74640
tgctccagct cagccccagg ggacacagat gcactttggg ggtgagggca ggtaatgact 74700
ccatcgcacc ctcagttcag ctggacagag gctcaggtga ccccagcctt cactgtctcc 74760
cgctctccag gagcttatct tcgccccatc tcccaaataa gtgggccctt gtgctgtgag 74820
gaagaccaaa gcctcaggga agataagaga tatggagatg ggagggggag gacaaggggc 74880
agagagtagg gtctagctgg ctatctctgg ccttactaac acccccctgg aggcatgccc 74940
cttttctcca gcacacaagc acattggggc acctggaaat attggttcca ggctcctgtt 75000
ctctggactt cagatcctgg gggagcccct cccccccctg aatccctggc ttagctacct 75060
tcctgcctgt gcacctaaaa acctcaggtc agaactagga aaagagtttt gtttttattt 75120
ttttgaaatg gagtctcgtt ctgtcgccca ggctgaggtg cagtagtgca atctccgctc 75180
actacaacct ccactccctg gggctcaagc gatcctccca cctcagccgc cgaagtagct 75240
gggactatag gtgtgtacca tcacacctgg ctaatttttg tattttttgt agacacaggg 75300
tttcgccatg ttgcccaggc tggtcttgaa ttcctgagct caagcaacct gccggcctcg 75360
gcctcccaaa gtactgggat tacacgcaga aggcaccatg cccaggctag atgtgtctta 75420
tcccaatcct ttggcaggca tgcagctcca caggcgattt cttcaagcag ctgaagtgtt 75480
tagccctcct gggttaagag ccagataagg agaaatccct ttcctaggtt tggaatgtgt 75540
tgtgaaaaaa aagagaaatc cctggctcct ggagctggtg ggagacaaga ttaagcaaac 75600
ctcccctgac atgtatccct ttgaccccaa gctctgcctc ctccctgacc acccatgccc 75660
tttcctttaa cttctcaaac agataccagg gcctaaactg ctttacctcc cctcctactg 75720
agtcaggtta ggtggtggga ggtcacccat ttccgagtta aaccaatgca atatgagtaa 75780
aacaaagtca tgtgggtatg tctggggtag agagaggggt agcaagttca tgtgtcctcc 75840
ttggtcacat atctcccaaa gctccgatcc ctgccatggg aagtggacag gaaacatgag 75900
gtcatgacct gcaggcatct ttactgcagc tctgccggcc tggaggggga gagggggagg 75960
aagaagtatg cgctgcacat ttctgaggct actgcatttg ctttcaaggc agaaatcttg 76020
ctctgagcag tcagcggctc cagtttgggc ccgataagga agttctccgt ggcctccctc 76080
aggcagagca gggaggaggc tgacattgcc agtctcttct ggggcccaag gcaggttgca 76140
ggagatccaa tcccatagac agctctgggc ctcttgcatt tgagtttttc agaattaaac 76200
tgcagtattt tggaaagca 76219

<210> 6
<211> 21
<212> DNA
<213> artificial

<220>
<223> siRNA sense sequence

<400> 6
ggcggauaug agcagcgaat t 21

<210>  7
<211>  21
<212>  DNA
<213>  artificial

<220>
<223>  siRNA sense sequence

<400>  7
guaugacauc accaacaaat t                                                  21


<210>  8
<211>  21
<212>  DNA
<213>  artificial

<220>
<223>  siRNA sense sequence

<400>  8
gaagcgucac ccaugcuuat t                                                  21


<210>  9
<211>  21
<212>  DNA
<213>  artificial

<220>
<223>  siRNA sense sequence

<400>  9
gagacuaugu agagucccat t                                                  21